# EUROPEAN PATENT APPLICATION

(11) **EP 2 196 544 A1**
(43) Date of publication of application: **16.06.2010**
(21) Application number: 09016057.3
(22) Date of filing: 21.11.2002
(51) Int. Cl.: C12Q 1/68, G01N 33/50, G01N 33/53, G01N 33/543

(54) **Kit for ligation detection assays using codeable labels**

(30) Priority: 21.11.2001 US 332519 P; 31.05.2002 US 384731 P
(62) Divisional of application: 02797138.1
(71) Applicant: Applied Biosystems, LLC, Carlsbad, CA 92008 (US)
(72) Inventor: Vann, Charles S., Burlingame, CA 94010 (US); Lao, Kai, Qin, Pleasanton, CA 94566 (US); Reed, Mark, Menlo Park, CA 94025 (US)
(74) Representative: Grund, Martin

(57) **Abstract**

The invention relates to kits for the detection of targets in a sample. In certain embodiments, methods for quantitating a target are provided. In certain embodiments, the methods comprise forming a reaction mixture comprising: a sample possibly containing the target; a codeable label; one or more target-specific probes, wherein each target-specific probe binds specifically to the target under selective binding conditions; and a separating moiety. In certain embodiments, the methods further comprise treating the reaction mixture with a ligase under reaction conditions such that a detectable complex is produced when the target is present, and such that a detectable complex is not produced when the target is absent, and wherein the detectable complex comprises the codeable label, the target-specific probe, and the separating moiety. In certain embodiments, the methods further comprise separating the detectable complex from codeable labels that are not included in the detectable complex, and quantitating the target by counting the number of codeable labels.

## Description

### Priority Data

This application claims the benefit of priority of U.S. Provisional Application Nos. 60/332.519, filed November 21, 2001, and 60/384,731, filed May 31, 2002. Application Nos. 60/332,519 and 60/384,731 are incorporated by reference herein in their entirety for any purpose.

### Field of the Invention

The invention relates to methods and compositions for the detection of targets in a sample.

### Background

The detection of the presence or absence of one or more target sequences in a sample containing one or more target sequences is commonly practiced. For example, the detection of cancer and many infectious diseases, such as AIDS and hepatitis, routinely includes screening biological samples for the presence or absence of diagnostic nucleic acid sequences. Also, detecting the presence or absence of nucleic acid sequences is often used in forensic science, paternity testing, genetic counseling, and organ transplantation.

### Summary of the Invention

In certain embodiments, methods for quantitating a target are provided. In certain embodiments, the methods comprise forming a reaction mixture comprising: a sample possibly containing the target; a codeable label; one or more target-specific probes, wherein each target-specific probe binds specifically to the target under selective binding conditions; and a separating moiety. In certain embodiments, the methods further comprise treating the reaction mixture under reaction conditions such that a detectable complex is produced when the target is present, and such that a detectable complex is not produced when the target is absent, and wherein the detectable complex comprises the codeable label, the target-specific probe, and the separating moiety. In certain embodiments, the methods further comprise separating the detectable complex from codeable labels that are not included in the detectable complex, and quantitating the target by counting the number of codeable labels.

In certain embodiments, methods for quantitating at least two different particular targets are provided. In certain embodiments, the methods comprise forming a reaction mixture comprising: a sample possibly containing two or more different particular targets; a different codeable label specific for each different particular target; one or more different target-specific probes specific for each different particular target that bind specifically to the target under selective binding conditions; and a separating moiety. In certain embodiments, the methods further comprise treating the reaction mixture under reaction conditions such that when a particular target is present, a detectable complex is produced, which comprises the codeable label specific for the particular target, the target-specific probe specific for the particular target, and the separating moiety, and when a particular target is absent, a detectable complex is not produced. In certain embodiments, the methods further comprise separating any detectable complexes produced from codeable labels that are not included in the detectable complex, and quantitating each of the different particular targets by counting the number of codeable labels specific for each of the different particular targets.

In certain embodiments, methods for quantitating at least two different target nucleic acid sequences in a sample are provided. In certain embodiments, the methods comprise forming a ligation reaction mixture by combining the sample with a different probe set specific for each of the at least two different target nucleic acid sequences. In certain embodiments, each probe set comprises (a) at least one separating bead, comprising a magnetic particle and a first target-specific probe, and (b) at least one detecting bead, comprising a codeable label, and a second target-specific probe; wherein the target-specific probes in each set are suitable for ligation together when hybridized adjacent to one another on a complementary target sequence. In certain embodiments, the methods further comprise subjecting the ligation reaction mixture to a ligation reaction, wherein adjacently hybridizing complementary target-specific probes are ligated to one another to form a ligation product comprising the separating bead and the detecting bead. In certain embodiments, the methods further comprise separating any ligation product from unligated separating and detecting beads. In certain embodiments, the methods further comprise quantitating each of the at least two different target nucleic acid sequences by counting the number of codeable labels.

In certain embodiments, kits for detecting target nucleic acid sequences in a sample are provided. In certain embodiments, the kits comprise a different bead set specific for each of the target nucleic acid sequences. In certain embodiments, each different bead set comprises (a) at least one separating bead, comprising a magnetic particle, a first codeable label comprising two or more labels, and a first target-specific probe, wherein the first codeable label is specific for the first target-specific probe, and (b) at least one detecting bead, comprising a second codeable label comprising a set of two or more labels, and a second target-specific probe, wherein the second codeable label is specific for the second target-specific probe; and wherein the first codeable label is detectably different from the second codeable label. In certain embodiments, the target-specific probes in each set are suitable for ligation together when hybridized adjacent to one another on a complementary target sequence.

### Brief Description of the Figures

Figure 1 illustrates a probe set according to certain embodiments of the invention.
Figure 2 illustrates methods for differentiating between two potential alleles in a target locus using certain embodiments of the Invention.
Fig. 2(A) shows: (i) two different probe sets that have different first target-specific probes, A and B, that differ in their pivotal complement (T on the A probe and C on the B probe), and that have the same second target-specific probe, Z, and (ii) a target sequence, comprising pivotal nucleotide A.
Fig. 2(B) shows the three target-specific probes annealed to the target. The sequence-specific portion of probe A is fully complementary with the 3' target region including the pivotal nucleotide. The pivotal complement of probe B is not complementary with the 3' target region. The sequence-specific portion of probe B, therefore, contains a base-pair mismatch at the 3' end. The sequence-specific portion of probe Z is fully complementary to the 5' target region.
Fig. 2(C) shows ligation of target-specific probes A and Z to form ligation product A-Z. Probes B and Z are not ligated together to form a ligation product due to the mismatched pivotal complement on probe B.
Fig. 2(d) shows denaturing the double-stranded molecules to release the A-Z ligation product and unligated probes B and Z.
Figure 3 illustrates certain potential binary and ternary codes using two colors of labels according to certain embodiments.
Figure 4 illustrates certain combinations of sets of labels (codes) when one uses a two color binary code with a probe set according to certain embodiments. Figure 4 also depicts the number of potential probe set codes according to certain embodiments when two ternary colors, 10 binary colors, or 6 ternary colors are used.
Figure 5 depicts exemplary alternative splicing.
Figure 6 depicts certain embodiments for detecting splice variants.
Figure 7 illustrates certain exemplary embodiments in which a first target specific probe and a second target specific probe are ligated after hybridizing to a target molecule in a sample.
Figure 8 illustrates certain exemplary embodiments in which separating moieties are separated from codeable labels and detectable complexes.
Figure 9 illustrates certain embodiments of detecting of detectable complexes that have been separated from the sample.
Figure 10 illustrates certain exemplary embodiments in which separating moieties are separated from codeable labels and detectable complexes.
Figure 11 illustrates certain exemplary embodiments in which ligated detectable complexes are separated from unligated codeable labels.
Figure 12 illustrates certain exemplary embodiments in which ligated detectable complexes are detected within the same vessel as the sample and ligation reaction.
Figure 13 illustrates certain exemplary embodiments in which a groove is included on the inner surface of the detection vessel for assisting in aligning ligated detectable complexes for detection.
Figure 14(a) illustrates a probe set according to certain embodiments of the invention.
Figure 14(b) illustrates two probe sets, ligation of the probe sets, and detection of probe sets according to certain embodiments of the invention.
Figure 14(c) illustrates a probe set according to certain embodiments of the invention.
Figure 15 depicts results from a Taqman^{™} analysis of ligated detectable complexes comprising beads and ligation products that do not comprise beads.
Figure 16 shows photographs of detectable complexes obtained after ligation reactions.
Figure 17 depicts the results of Taqman analyses of ligated detectable complexes produced in different concentrations of target molecules.
Figure 18 illustrates certain exemplary embodiments of separation of unpaired nonmagnetic beads from magnetic beads and detectable complexes by continuous flow, and a subsequent counting of detectable complexes by flow cytometry.
Figure 19 illustrates certain exemplary embodiments of separation of unpaired nonmagnetic beads from magnetic beads and detectable complexes by continuous flow, removal of unpaired magnetic beads by the difference in drag between detectable complexes and unpaired magnetic beads, and subsequent counting of detectable complexes by flow cytometry.
Figure 20 illustrates certain exemplary embodiments of separation of unpaired nonmagnetic beads from magnetic beads and detectable complexes by continuous flow, separation of unpaired magnetic beads by size filtration, and subsequent counting of detectable complexes by flow cytometry.
Figure 21 illustrates certain exemplary embodiments of a separation method employing magnetic beads and biotin-coated beads.
Figure 22 illustrates certain exemplary embodiments of a ligation reaction employing probes comprising addressable portions.
Figure 23 illustrates certain exemplary embodiments in which a ligation product is attached to beads using hairpin structures.
Figure 24 illustrates certain exemplary embodiments in which a ligation product is attached to beads using linking oligonucleotides.
Figure 25 illustrates certain exemplary embodiments of an oligonucleotide ligation (OLA) assay with a biotin molecule.
Figure 26 illustrates certain exemplary embodiments of a codeable label attached to an oligonucleotide that hybridizes to a ligation product.
Figure 27 illustrates certain exemplary embodiments of separating detectable complexes from codeable labels not in detectable complexes.
Figure 28 illustrates certain exemplary embodiments of separating detectable complexes from codeable labels not in detectable complexes using a tube within a tube.
Figure 29 illustrates certain exemplary embodiments of a codeable label attached to a hairpin structure that hybridizes and ligates to a ligation product.

### Detailed Description of Exemplary Embodiments

It is to be understood that both the foregoing general description and the following detailed description are exemplary and explanatory only and are not restrictive of the invention, as claimed. In this application, the use of the singular includes the plural unless specifically stated otherwise. In this application, the use of "or" means "and/or" unless stated otherwise. Furthermore, the use of the term "including", as well as other forms, such as "includes" and "included," is not limiting. Also, the use of the term "portion" may include part of a moiety or the entire moiety.

The section headings used herein are for organizational purposes only and are not to be construed as limiting the subject matter described. All documents, or portions of documents, cited in this application, including but not limited to patents, patent applications, articles, books, and treatises, are hereby expressly incorporated by reference in their entirety for any purpose.

### Definitions and Terms

The term "nucleotide base", as used herein, refers to a substituted or unsubstituted aromatic ring or rings. In certain embodiments, the aromatic ring or rings contain at least one nitrogen atom. In certain embodiments, the nucleotide base is capable of forming Watson-Crick and/or Hoogsteen hydrogen bonds with an appropriately complementary nucleotide base. Exemplary nucleotide bases and analogs thereof include, but are not limited to, naturally occurring nucleotide bases adenine, guanine, cytosine, uracil, thymine, and analogs of the naturally occurring nucleotide bases, e.g., 7-deazaadenine, 7-deazaguanine, 7-deaza-8-azaguanine, 7-deaza-8-azaadenine, N6 -Δ2 -isopentenyladenine (6iA), N6 -Δ2 -isopentenyl-2-methylthioadenine (2ms6iA), N2 -dimethylguanine (dmG), 7-methylguanine (7mG), inosine, nebularine, 2-aminopurine, 2-amino-6-chloropurine, 2,6-diaminopurine, hypoxanthine, pseudouridine, pseudocytosine, pseudoisocytosine, 5-propynylcytosine, isocytosine, isoguanine, 7-deazaguanine, 2-thiopyrimidine, 6-thioguanine, 4-thiothymine, 4-thiouracil, *O*⁶-methylguanine, *N*⁶-methyladenine, *O*⁴-methylthymine, 5,6-dihydrothymine, 5,6-dihyorouracil, pyrazolo[3.4-D]pyrimidines (see, e.g., U.S. Patent Nos. 6,143,877 and 6,127,121 and PCT published application WO 01/38584), ethenoadenine, indoles such as nitroindole and 4-methylindole, and pyrroles such as nitropyrrole. Certain exemplary nucleotide bases can be found, e.g., in Fasman, 1989, Practical Handbook of Biochemistry and Molecular Biology, pp. 385-394, CRC Press, Boca Raton, Fla., and the references cited therein.

The term "nucleotide", as used herein, refers to a compound comprising a nucleotide base linked to the C-1' carbon of a sugar, such as ribose, arabinose, xylose, and pyranose, and sugar analogs thereof. The term nucleotide also encompasses nucleotide analogs. The sugar may be substituted or unsubstituted. Substituted ribose sugars include, but are not limited to, those riboses in which one or more of the carbon atoms, for example the 2'-carbon atom, is substituted with one or more of the same or different Cl, F, -R, -OR, -NR₂ or halogen groups, where each R is independently H, C₁-C₆ alkyl or C₅-C₁₄ aryl. Exemplary riboses include, but are not limited to, 2'-(C1 -C6)alkoxyribose, 2'-(C5 -C14)aryloxyribose, 2',3'-didehydroribose, 2'-deoxy-3'-haloribose, 2'-deoxy-3'-fluororibose, 2'-deoxy-3'-chlororibose, 2'-deoxy-3'-aminoribose, 2'-deoxy-3'-(C1-C6)alkylribose, 2'-deoxy-3'-(C1-C6)alkoxyribose and 2'-deoxy-3'-(C5-C14)aryloxyribose, ribose, 2'-deoxyribose, 2',3'-dideoxyribose, 2'-haloribose, 2'-fluororibose, 2'-chlororibose, and 2'-alkylribose, e.g., 2'-O-methyl, 4'-α-anomeric nucleotides, 1'-α-anomeric nucleotides, 2'-4'- and 3'-4'-linked and other "locked" or "LNA", bicyclic sugar modifications (see, e.g., PCT published application nos. WO 98/22489, WO 98/39352;, and WO 99/14226). Exemplary LNA sugar analogs within a polynucleotide include, but are not limited to, the structures: 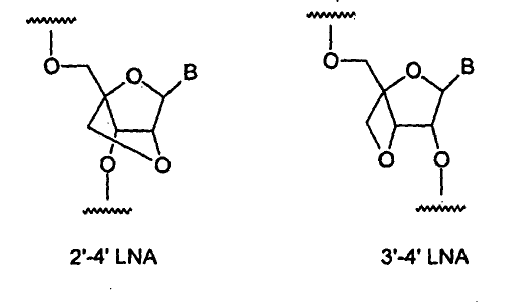 where B is any nucleotide base.

Modifications at the 2'- or 3'-position of ribose include, but are not limited to, hydrogen, hydroxy, methoxy, ethoxy, allyloxy, isopropoxy, butoxy, isobutoxy, methoxyethyl, alkoxy, phenoxy, azido, amino, alkylamino, fluoro, chloro and bromo. Nucleotides include, but are not limited to, the natural D optical isomer, as well as the L optical isomer forms (see, e.g., Garbesi (1993) Nucl. Acids Res. 21:4159-65; Fujimori (1990) J. Amer. Chem. Soc. 112:7435; Urata, (1993) Nucleic Acids Symposium Ser. No. 29:69-70). When the nucleotide base is purine, e.g. A or G, the ribose sugar is attached to the N⁹-position of the nucleotide base. When the nucleotide base is pyrimidine, e.g. C, T or U, the pentose sugar is attached to the N¹-position of the nucleotide base, except for pseudouridines, in which the pentose sugar is attached to the C5 position of the uracil nucleotide base (see, e.g., Kornberg and Baker, (1992) DNA Replication, 2nd Ed., Freeman, San Francisco, CA).

One or more of the pentose carbons of a nucleotide may be substituted with a phosphate ester having the formula: 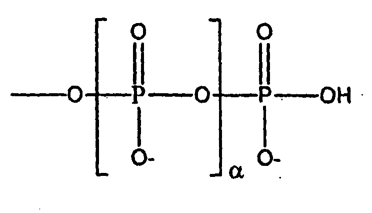 where α is an integer from 0 to 4. In certain embodiments, α is 2 and the phosphate ester is attached to the 3'- or 5'-carbon of the pentose. In certain embodiments, the nucleotides are those in which the nucleotide base is a purine, a 7-deazapurine, a pyrimidine, or an analog thereof. "Nucleotide 5'-triphosphate" refers to a nucleotide with a triphosphate ester group at the 5' position, and are sometimes denoted as "NTP", or "dNTP" and "ddNTP" to particularly point out the structural features of the ribose sugar. The triphosphate ester group may include sulfur substitutions for the various oxygens, e.g. α-thio-nucleotide 5'-triphosphates. For a review of nucleotide chemistry, see: Shabarova, Z. and Bogdanov, A. Advanced Organic Chemistry of Nucleic Acids, VCH, New York, 1994.

The term "nucleotide analog", as used herein, refers to embodiments in which the pentose sugar and/or the nucleotide base and/or one or more of the phosphate esters of a nucleotide may be replaced with its respective analog. In certain embodiments, exemplary pentose sugar analogs are those described above. In certain embodiments, the nucleotide analogs have a nucleotide base analog as described above. In certain embodiments, exemplary phosphate ester analogs include, but are not limited to, alkylphosphonates, methylphosphonates, phosphoramidates, phosphotriesters, phosphorothioates, phosphorodithioates, phosphoroselenoates, phosphorodiselenoates, phosphoroanilothioates, phosphoroanilidates, phosphoroamidates, boronophosphates, etc., and may include associated counterions.

Also included within the definition of "nucleotide analog" are nucleotide analog monomers which can be polymerized into polynucleotide analogs in which the DNA/RNA phosphate ester and/or sugar phosphate ester backbone is replaced with a different type of intemucleotide linkage. Exemplary polynucleotide analogs include, but are not limited to, peptide nucleic acids, in which the sugar phosphate backbone of the polynucleotide is replaced by a peptide backbone.

As used herein, the terms "polynucleotide", "oligonucleotide", and "nucleic acid" are used interchangeably and mean single-stranded and double-stranded polymers of nucleotide monomers, including 2'-deoxyribonucleotides (DNA) and ribonucleotides (RNA) linked by internucleotide phosphodiester bond linkages, or internucleotide analogs, and associated counter ions, e.g., H⁺, NH₄⁺, trialkylammonium, Mg²⁺, Na⁺ and the like. A nucleic acid may be composed entirely of deoxyribonucleotides, entirely of ribonucleotides, or chimeric mixtures thereof. The nucleotide monomer units may comprise any of the nucleotides described herein, including, but not limited to, naturally occuring nucleotides and nucleotide analogs. Nucleic acids typically range in size from a few monomeric units, e.g. 5-40 when they are sometimes referred to in the art as oligonucleotides, to several thousands of monomeric nucleotide units. Unless denoted otherwise, whenever a nucleic acid sequence is represented, it will be understood that the nucleotides are in 5' to 3' order from left to right and that "A" denotes deoxyadenosine or an analog thereof, "C" denotes deoxycytidine or an analog thereof, "G" denotes deoxyguanosine or an analog thereof, and "T" denotes thymidine or an analog thereof, unless otherwise noted.

Nucleic acids include, but are not limited to, genomic DNA, cDNA, hnRNA, mRNA, rRNA, tRNA, fragmented nucleic acid, nucleic acid obtained from subcellular organelles such as mitochondria or chloroplasts, and nucleic acid obtained from microorganisms or DNA or RNA viruses that may be present on or in a biological sample.

Nucleic acids may be composed of a single type of sugar moiety, e.g., as in the case of RNA and DNA, or mixtures of different sugar moieties, e.g., as in the case of RNA/DNA chimeras. In certain embodiments, nucleic acids are ribopolynucleotides and 2'-deoxyribopolynucleotides according to the structural formulae below: 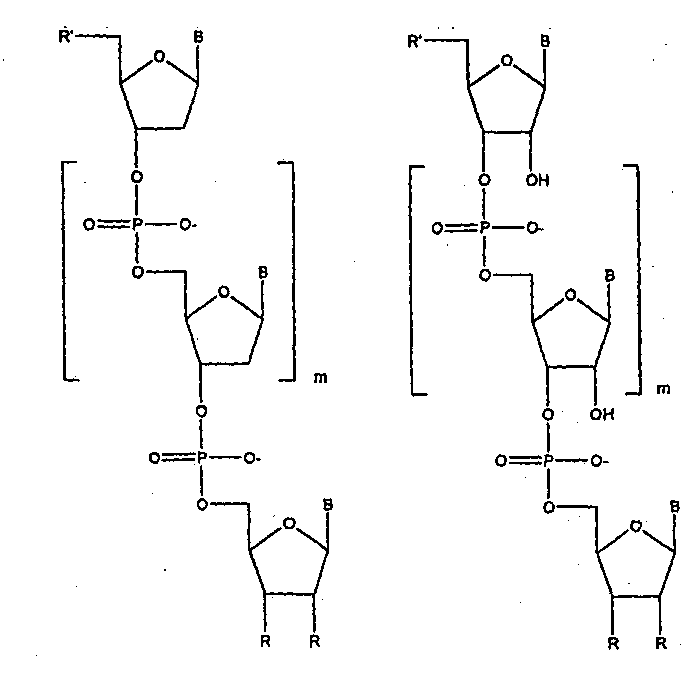 wherein each B is independently the base moiety of a nucleotide, e.g., a purine, a 7-deazapurine, a pyrimidine, or an analog nucleotide; each m defines the length of the respective nucleic acid and can range from zero to thousands, tens of thousands, or even more; each R is independently selected from the group comprising hydrogen, halogen, --R", -OR", and -NR"R", where each R" is independently (C1 -C6) alkyl or (C5 -C14) aryl, or two adjacent Rs are taken together to form a bond such that the ribose sugar is 2',3'-didehydroribose; and each R' is independently hydroxyl or 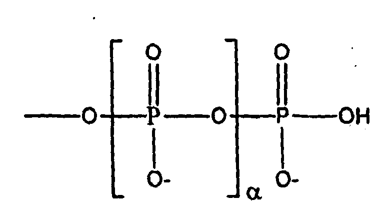 where α is zero, one or two.

In certain embodiments of the ribopolynucleotides and 2'-deoxyribopolynucleotides illustrated above, the nucleotide bases B are covalently attached to the C1' carbon of the sugar moiety as previously described.

The terms "nucleic acid", "polynucleotide", and "oligonucleotide" may also include nucleic acid analogs, polynucleotide analogs, and oligonucleotide analogs. The terms "nucleic acid analog", "polynucleotide analog" and "oligonucleotide analog" are used interchangeably and, as used herein, refer to a nucleic acid that contains at least one nucleotide analog and/or at least one phosphate ester analog and/or at least one pentose sugar analog. Also included within the definition of nucleic acid analogs are nucleic acids in which the phosphate ester and/or sugar phosphate ester linkages are replaced with other types of linkages, such as N-(2-aminoethyl)-glycine amides and other amides (see, e.g., Nielsen et al., 1991, Science 254: 1497-1500; WO 92/20702; U.S. Pat. No. 5,719,262; U.S. Pat. No. 5,698,685;); morpholinos (see, e.g., U.S. Pat. No. 5,698,685; U.S. Pat. No. 5,378,841; U.S. Pat. No. 5,185,144); carbamates (see, e.g., Stirchak & Summerton, 1987, J. Org. Chem. 52: 4202); methylene(methylimino) (see, e.g., Vasseur et al., 1992, J. Am. Chem. Soc. 114: 4006); 3'-thioformacetals (see, e.g., Jones et al., 1993, J. Org. Chem. 58: 2983); sulfamates (see, e.g., U.S. Pat. No. 5,470,967); 2-aminoethylglycine, commonly referred to as PNA (see, e.g., Buchardt, WO 92/20702; Nielsen (1991) Science 254:1497-1500); and others (see, e.g., U.S. Pat. No. 5,817,781; Frier & Altman, 1997, Nucl. Acids Res. 25:4429 and the references cited therein). Phosphate ester analogs include, but are not limited to, (i) C₁-C₄ alkylphosphonate, e.g. methylphosphonate; (ii) phosphoramidate; (iii) C₁-C₆ alkylphosphotriester; (iv) phosphorothioate; and (v) phosphorodithioate.

The terms "annealing" and "hybridization" are used interchangeably and mean the base-pairing interaction of one nucleic acid with another nucleic acid that results in formation of a duplex, triplex, or other higher-ordered structure. In certain embodiments, the primary interaction is base specific, e.g., A/T and G/C, by Watson/Crick and Hoogsteen-type hydrogen bonding. In certain embodiments, base-stacking and hydrophobic interactions may also contribute to duplex stability.

The term "variant" as used herein refers to any alteration of a protein, including, but not limited to, changes in amino acid sequence, substitutions of one or more amino acids, addition of one or more amino acids, deletion of one or more amino acids, and alterations to the amino acids themselves. In certain embodiments, the changes involve conservative amino acid substitutions. Conservative amino acid substitution may involve replacing one amino acid with another that has, e.g., similar hydorphobicity, hydrophilicity, charge, or aromaticity. In certain embodiments, conservative amino acid substitutions may be made on the basis of similar hydropathic indices. A hydropathic index takes into account the hydrophobicity and charge characteristics of an amino acid, and in certain embodiments, may be used as a guide for selecting conservative amino acid substitutions. The hydropathic index is discussed, e.g., in Kyte et al., J. Mol. Biol., 157:105-131 (1982). It is understood in the art that conservative amino acid substitutions may be made on the basis of any of the aforementioned characteristics.

Alterations to the amino acids may include, but are not limited to, glycosylation, methylation, phosphorylation, biotinylation, and any covalent and noncovalent additions to a protein that do not result in a change in amino acid sequence. "Amino acid" as used herein refers to any amino acid, natural or nonnatural, that may be incorporated, either enzymatically or synthetically, into a polypeptide or protein.

As used herein, an "affinity set" is a set of molecules that specifically bind to one another. Affinity sets include, but are not limited to, biotin and avidin, biotin and streptavidin, receptor and ligand, antibody and ligand, antibody and antigen, and a polynucleotide sequence and its complement. In certain embodiments, affinity sets that are bound may be unbound. For example, a polynucleotide sequences that are hybridized may be denatured, and biotin bound to streptavidin may be heated and become unbound.

A "target" refers to any material that can be distinguished by a probe. Targets may include both naturally occurring and synthetic molecules.

In certain embodiments, targets may include nucleic acid sequences. In certain embodiments, target nucleic acid sequences may include RNA and DNA. Exemplary RNA target sequences include, but are not limited to, mRNA, rRNA, tRNA, viral RNA, and variants of RNA, such as splicing variants. Exemplary DNA target sequences include, but are not limited to, genomic DNA, plasmid DNA, phage DNA, nucleolar DNA, mitochondrial DNA, and chloroplast DNA.

In certain embodiments, nucleic acid sequences include, but are not limited to, cDNA, yeast artificial chromosomes (YAC's), bacterial artificial chromosomes (BAC's), other extrachromosomal DNA, and nucleic acid analogs. Exemplary nucleic acid analogs include, but are not limited to, LNAs, PNAs, PPG's, and other nucleic acid analogs discussed below.

A variety of methods are available for obtaining a target nucleic acid sequence for use with the compositions and methods of the present invention. When the nucleic acid target is obtained through isolation from a biological matrix, certain isolation techniques include (1) organic extraction followed by ethanol precipitation, e.g., using a phenol/chloroform organic reagent (e.g., Ausubel et al., eds., Current Protocols in Molecular Biology Volume 1, Chapter 2, Section I, John Wiley & Sons, New York (1993)), preferably using an automated DNA extractor, e.g., the Model 341 DNA Extractor available from PE Applied Biosystems (Foster City, CA); (2) stationary phase adsorption methods (e.g., Boom et al., U.S. Patent No. 5,234,809; Walsh et al., Biotechniques 10(4): 506-513 (1991)); and (3) salt-induced DNA precipitation methods (e.g., Miller et al., Nucleic Acids Research,16(3): 9-10 (1988)), such precipitation methods being typically referred to as "salting-out" methods. In certain embodiments, the above isolation methods may be preceded by an enzyme digestion step to help eliminate unwanted protein from the sample, e.g., digestion with proteinase K, or other like proteases.

In certain embodiments, target nucleic acid sequences include, but are not limited to, amplification products, ligation products, transcription products, reverse transcription products, primer extension products, methylated DNA, and cleavage products. In certain embodiments, the target nucleic acid sequences may be produced by whole genome amplification. In certain embodiments, the target nucleic acid sequences may be produced by isothermal amplification and/or ligation.

In certain embodiments, nucleic acids in a sample may be subjected to a cleavage procedure such as the cleavage procedure in an Invader^{™} assay (as exemplified, e.g., in U.S. Patent Nos. 5,846,717; 5,985,557; 5,994,069; 6,001;567; and 6,090,543). Such procedures produce a cleavage product when a nucleic acid of interest is present in a sample. In certain embodiments, the target may be such a cleavage product. Briefly, the cleavage procedure may employ two nucleic acid oligonucleotides that are designed to be complementary to the nucleic acid in the sample. A first oligonucleotide comprises a 5' portion that does not complement the nucleic acid in the sample, contiguous with a 3' portion that does complement the nucleic acid in the sample. A second oligonucleotide complements the nucleic acid in the sample in a region of the nucleic acid in the sample that is 3' of the region complemented by the first oligonucleotide, and includes a complementary or non-complementary portion that slightly overlaps with the region complemented by the first oligonucleotide. Hybridization of the two oligonucleotides to the nucleic acid in the sample causes a portion of the first oligonucleotide to be cleaved, often in the presence of an enzyme. The cleavage product is typically the 5' portion of the first oligonucleotide that does not complement the nucleic acid in the sample, and that portion of the complementary region that overlaps with the second oligonucleotide. This cleavage product comprises a known nucleic acid sequence. In certain embodiments, such cleavage products may be targets.

Different target nucleic acid sequences may be different portions of a single contiguous nucleic acid or may be on different nucleic acids. Different portions of a single contiguous nucleic acid may overlap.

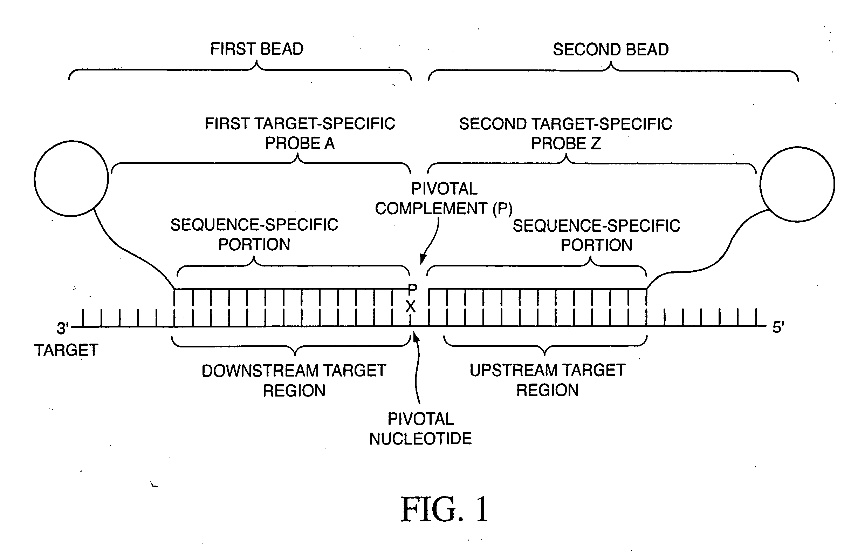

In certain embodiments, a target nucleic acid sequence comprises an upstream or 5' region, a downstream or 3' region, and a "pivotal nucleotide" located between the upstream region and the downstream region (see, e.g., Figure 1). The pivotal nucleotide is the nucleotide being detected by the probe set and may represent, for example, without limitation, a single polymorphic nucleotide in a multiallelic target locus.

The person of ordinary skill will appreciate that while a target nucleic acid sequence is typically described as a single-stranded molecule, the opposing strand of a double-stranded molecule comprises a complementary sequence that may also be used as a target sequence.

Other targets include, but are not limited to, peptide sequences. Peptides sequences include, but are not limited to, proteins, fragments of proteins, and other segments of amino acids. In certain embodiments, peptide target sequences include, but are not limited to, different peptide alleles (similar peptides with different amino acids) and different peptide conformations (similar proteins with different secondary and tertiary structures). Other naturally occurring targets include, but are not limited to, hormones and other signal molecules, such as hormones and other steroid-type molecules.

In certain embodiments, targets include, but are not limited to, synthetic peptides, pharmaceuticals, and other organic small molecules.

### Probes

The term "probe" or "target-specific probe" is any moiety that comprises a portion that can specifically bind a target. Probes may include, but are not limited to, nucleic acids, peptides, and other molecules that can specifically bind a target in a sample. Such specific binding includes, but is not limited to, hybridization between nucleic acid molecules, antibody-antigen interactions, interactions between ligands and receptors, and interactions between aptomers and proteins.

In certain embodiments, a probe comprises a nucleic acid sequence-specific portion that is designed to hybridize in a sequence-specific manner with a complementary region on a selected target nucleic acid sequence. In certain embodiments, the sequence-specific portion of the probe may be specific for a particular sequence, or alternatively, may be degenerate, e.g., specific for a set of sequences. A probe for a target peptide may comprise an antibody, as a non-limiting example.

In certain embodiments, probes comprise aptomers, which are nucleic acids that specifically bind to certain peptide sequences. In certain embodiments, probes comprise peptides. Such peptides include, but are not limited to, antibodies and receptor molecules. In certain embodiments, probes comprise antibodies directed to specific target peptide antigens.

In certain embodiments, probes may include other members of unique binding pairs, such as streptavidin/biotin binding pairs, and affinity binding chemicals available from Prolinx^{™} (Bothell, WA) as exemplified, e.g., by U.S. Patent Nos. 5,831, 046; 5,852,178; 5,859,210; 5,872,224; 5,877,297; 6,008,406; 6,013,783; 6,031,17; and 6,075,126.

A "probe set" according to the present invention is a group of two or more probes designed to detect at least one target. As a non-limiting example, a probe set may comprise two nucleic acid probes designed to hybridize to a target such that, when the two probes are hybridized to the target adjacent to one another, they are suitable for ligation together.

When used in the context of the present invention, "suitable for ligation" refers to at least one first target-specific probe and at least one second target-specific probe, each comprising an appropriately reactive group. Exemplary reactive groups include, but are not limited to, a free hydroxyl group on the 3' end of the first probe and a free phosphate group on the 5' end of the second probe, phosphorothioate and tosylate or iodide, esters and hydrazide, RC(O)S', haloalkyl, RCH₂S and α-haloacyl, thiophosphoryl and bromoacetoamido groups, and S-pivaloyloxymethyl-4-thiothymidine. Additionally, in certain embodiments, the first and second target-specific probes are hybridized to the target sequence such that the 3' end of the first target-specific probe and the 5' end of the second target-specific probe are immediately adjacent to allow ligation.

### Codeable Labels

The term "label" refers to any molecule or set of molecules that can provide a detectable signal or interacts with a second molecule or other member of the set of molecules to provide a detectable signal - either provided by the first molecule or provided by the second molecule, e.g., FRET (Fluorescent Resonance Energy Transfer). Use of labels can be accomplished using any one of a large number of known techniques employing known labels, linkages, linking groups, reagents, reaction conditions, and analysis and purification methods. Labels include, but are not limited to, light-emitting or light-absorbing compounds which generate or quench a detectable fluorescent, chemiluminescent, or bioluminescent signal (see, e.g., Kricka, L. in Nonisotopic DNA Probe Techniques (1992), Academic Press, San Diego, pp. 3-28). Fluorescent reporter dyes useful as labels include, but are not limited to, fluoresceins (see, e.g., U.S. Patent Nos. 5,188,934; 6,008,379; and 6,020,481), rhodamines (see, e.g., U.S. Patent Nos. 5,366,860; 5,847,162; 5,936,087; 6,051,719; and 6,191,278), benzophenoxazines (see, e.g., U.S. Patent No. 6,140,500), energy-transfer fluorescent dyes, comprising pairs of donors and acceptors (see, e.g., U.S. Patent Nos. 5,863,727; 5,800,996; and 5,945,526), and cyanines (see, e.g., Kubista, WO 97/45539), as well as any other fluorescent moiety capable of generating a detectable signal. Examples of fluorescein dyes include, but are not limited to, 6-carboxyfluorescein; 2',4',1,4,-tetrachlorofluorescein; and 2',4',5',7',1,4-hexachlorofluorescein.

Other exemplary labels include, but are not limited to, luminescent molecules that emit light, and molecules that can be involved in luminescent reactions, such as luciferin-luciferase reactions, as a non-limiting example. Labels also include, but are not limited to, chemiluminescent and electroluminescent molecules and reactions. As a non-limiting example, chemiluminescent labels may be exposed to film. Development of the film indicates whether or not targets are present in the sample or the quantity of the targets in the sample.

Other exemplary labels include, but are not limited to, donor-acceptor interactions, in which a donor molecule emits energy that is detected by an acceptor molecule. The acceptor molecule then emits a detectable signal.

Other exemplary labels include, but are not limited to, molecules that are involved in infrared photon release.

Labels also include, but are not limited to, quantum dots. "Quantum dots" refer to semiconductor nanocrystalline compounds capable of emitting a second energy in response to exposure to a first energy. Typically, the energy emitted by a single quantum dot always has the same predictable wavelength. Exemplary semiconductor nanocrystalline compounds include, but are not limited to, crystals of CdSe, CdS, and ZnS. Suitable quantum dots according to certain embodiments are described, e.g., in U.S. Pat. Nos. 5,990,479 and 6,207,392 B1, and in "Quantum-dottagged microbeads for multiplexed optical coding of biomolecules," Han et al., Nature Biotechnology, 19:631-635 (2001).

Labels of the present invention also include phosphors and radioisotopes. Radioisotopes may be directly detected, or may excite a fluorophore that emits a wavelength of light that is then detected. Phosphor particles may be excited by an infrared light (approximately around 980 nm) but emit signals within the visible spectrum, thus significantly reducing or eliminating background light.

Other examples of certain exemplary labels include particles with coded information, such as barcodes, and also include the microparticle tags described in U.S. Patent No. 4,053,433. Certain other non-radioactive labeling methods, techniques, and reagents are reviewed in: Non-Radioactive Labelling, A Practical Introduction, Garman, A.J. (1997) Academic Press, San Diego.

A class of labels effect the separation or immobilization of a molecule by specific or non-specific capture, for example biotin, digoxigenin, and other haptens (see, e.g., Andrus, A. "Chemical methods for 5' non-isotopic labeling of PCR probes and primers" (1995) in PCR 2: A Practical Approach, Oxford University Press, Oxford, pp. 39-54).

"Codeable label" refers to the one or more labels which is specific to a particular moiety. In certain embodiments the moiety is a target and/or a probe. In embodiments in which a codeable label comprises more than one label, the labels may be the same or different. Detection of a given codeable label indicates the presence of the moiety to which the codeable label is specific. The absence of a given codeable label indicates the absence of the moiety to which the codeable label is specific.

Codeable labels may be described as "detectably different," which means that they are distinguishable from one another by at least one detection method. Different codeable labels include, but are not limited to, one or more labels that emit light of different wavelengths, one or more labels that emit light of different intensities, one or more labels that emanate different numbers and/or patterns of signals, one or more labels that have different fluorescent decay lifetimes, one or more labels that have different spectral signatures, one or more labels that have different radioactive decay properties, one or more labels of different charge, and one or more labels of different size.

In certain embodiments, the number of codeable labels is counted, which refers to the actual counting of individual codeable labels. Counting the number of codeable labels is distinguishable from analog signal detection, where an aggregate level of signal from multiple labels is detected. Analog signal detection typically uses integration of signals from multiple labels of the same type to determine the number of such labels present in a sample. For example, analog detection typically provides an estimate of the number of labels of a given type by comparing the brightness or level of intensity of the signal in the test sample to the brightness or level of intensity of the signal in controls with known quantities of the given labels.

Counting, by contrast, is a digital detection system in which the number of individual codeable labels is actually counted. Thus, in certain embodiments, if 200 of the same codeable labels are present in a sample, each of those labels is actually counted. In certain embodiments, the number of labels counted may be within 20% of the actual number in the sample. In certain embodiments, the number of labels counted may be within 10% of the actual number in the sample. In certain embodiments, the number of labels counted may be within 50% of the actual number in the sample. In certain embodiments, a representative portion of those labels present in a sample are counted, and the total number of labels in the sample is determined by the number of labels counted in the representative portion. In contrast, to determine the number of labels in a sample with analog detection, the aggregate signal from the 200 labels is measured and compared to the aggregate signal from known quantities of labels.

In certain embodiments, the codeable labels and probes in a reaction are in sufficient excess of the target available that the number of codeable labels counted is representative to the number of targets present. In such embodiments, there is typically no more than one target bound to each codeable label.

In certain embodiments, since it involves the actual counting of codeable labels, digital detection may be less influenced by background "noise," or incidental light that may be interpreted as part of the aggregate signal in analog detection.

In certain embodiments, one may determine fine distinctions between different numbers of codeable labels in different samples by counting the number of codeable labels. In contrast, the aggregate signal from multiple labels in analog detection, in certain instances, may be affected by the variable amount of background signal in different_samples, which may obscure small differences in the number of labels in different samples.

In certain embodiments where two or more detectably different codeable labels are being detected in a sample, possible inaccuracies due to overlapping signals from detectably different codeable labels may be minimized by counting each of the detectably different codeable labels. In certain analog detection methods, part of the signal from one label may be detected as signal from another different label, which may result in an inaccurate reading. This may be particularly the case if the signals from the different labels have overlapping emission ranges. By counting the individual codeable labels, in certain embodiments, inaccuracies that may sometimes result may be minimized from analog detection where one measures the aggregate signal intensities from different labels.

In certain embodiments, the "codeable labels" are different sets of quantum dots that are specific for different target-specific probes (the different probes being specific for different target sequences), and the different sets of quantum dots are detectably different from one another.

Codeable labels may be attached directly to probes, or indirectly attached to other molecules that are then attached to probes. In certain embodiments, the codeable labels may be attached to a probe prior to being added to a sample, or may become attached to a probe during the course of a reaction that forms a detectable complex. In certain embodiments, codeable labels may be attached directly to a probe, or through a linking molecule, such as a chemical linkage group, or linking pair, such as a streptavidin-biotin pair.

In certain embodiments, labels are incorporated into beads, which may then be attached to probes. A "bead" refers to any material to which probes can be attached. Beads may be of any shape, including, but not limited to, spheres, rods, cubes, and bars. Beads may be made of any substance, including, but not limited to, silica glass and polymers. Beads may be any size. Certain non-limiting examples of beads include those described, e.g., in U.S. Pat. Nos. 4,499,052 (Fulwyler); 4,717,655 (Fulwyler); 3,957,741 (Rembaum, CalTech); 4,035,316 (Rembaum, CalTech); 4,105,598 (Rembaum, CalTech); 4,224,198 (Rembaum, CalTech); 4,326,008 (Rembaum, CalTech); 3,853,987 (Dreyer, CalTech); 4,108,972 (Dreyer, CalTech); 5,093,234 (Flow Cytometry Standards); 6,268,222 (Luminex); 5,326,692 (Molecular Probes); 5,573,909 (Molecular Probes); 5,723,218 (Molecular Probes); 5,786,219 (Molecular Probes); 5,028,545 (Soini); and 5,132,242 (Sau Cheung); as well as international application Publication Nos. WO 01/13119 (Luminex); WO 01/14589 (Luminex); WO 97/14028 (Luminex); WO 99/19515 (Luminex); WO 99/37814 (Luminex); WO 99/52708 (Luminex); WO 00/55363

(Amersham); WO 01/01141 (Amersham); WO 99/64867 (Amersham); and WO 94/11735 (Soini).

In certain embodiments, the beads comprise coated or uncoated particles comprising at least one of magnetic material, paramagnetic material, silica glass, polyacrylamide, polysaccharide, plastic, latex, polystyrene, and other polymeric substances.

Beads may comprise codeable labels, such as sets of quantum dots according to certain embodiments. Those skilled in the art are aware of suitable methods of obtaining beads with quantum dots. See, e.g., Han et al., Nature Biotechnology, 19:631-635 (2001), and U.S. Pat. Nos. 6,207,392 (Shuming Nie); 6,114,038 (Biocrystal); 6,261,779 (Biocrystal); 6,207,229 (Bawendi); 6,251,303 (Bawendi); 6,274,323 (Quantum Dot); 5,990,479 (Alivisatos); 6,207,392 (Alivisatos); international application Publication Nos. WO 00/29617 (Shuming Nie); WO 00/27365 (Blocrystal); WO 00/28089 (Biocrystal); WO 01/89585 (Biocrystal); WO 00/17642 (Bawendi); WO 00/17656 (Bawendi); WO 99/26299 (Bawendi); WO 00/68692 (Quantum Dot); WO 00/55631 (Alivisatos); and European Application No. 0 990 903 A1 (Bawendi). The quantum dots or other labels may be embedded in beads.

In certain embodiments, as a non-limiting example, quantum dots may be incorporated into cross-linked polymer beads. In certain embodiments, polystyrene beads may be synthesized using an emulsion of styrene (98% vol./vol.), divinylbenzene (1 % vol./vol.), and acrylic acid (1 % vol./vol.) at 70°C. In certain embodiments, the beads are then swelled in a solvent mixture containing 5% (vol./vol.) chloroform and 95 % (vol./vol.) propanol or butanol. In certain embodiments, a controlled amount of ZnS-capped CdSe quantum dots are added to the mixture. After incubation at room temperature, the embedding process is complete. In certain embodiments, the size of the beads may be controlled by the amount of a stabilizer (e.g., polyvinylpyrrolidone) used in the synthesis. In certain embodiments, a spherical bead 2 µm in diameter containing quantum dots that are 2-4 nm in diameter may contain tens of thousands of quantum dots.

The method of manufacturing beads discussed above may result in beads with varying numbers of quantum dots. Also, if one uses more than one color of quantum dot, one may obtain beads that have varying numbers of the different colors. In certain embodiments, after such bead preparation, the resulting beads are sorted by the relative number of quantum dots of each color in a given bead to obtain groups of identically labeled beads with distinct codeable labels. In certain embodiments, the sorting can be automated by machines, such as a Fluorescence Associated Cell Sorter (FACS) or other flow-cytometer type detection method that can distinguish between different codeable labels.

One of skill will appreciate that there are many methods of obtaining beads comprising probes. Such methods include, but are not limited to, attaching the probes to the beads using covalent bonding, UV crosslinking, and linking through an affinity set. As a non-limiting example, streptavidin molecules may be covalently attached to the carboxylic acid groups on the bead surface. Oligonucleotide probes may be biotinylated, then linked to the beads via the streptavidin molecules.

In certain embodiments, a bead contains an internal reference label. In certain embodiments, the internal reference label is detectably different than the codeable label. In certain embodiments, one may use an internal reference label to confirm the number of beads with codeable labels. For example, in certain embodiments, beads with different codeable labels will each include the same internal reference label that can be used to identify the presence of a single bead. In certain embodiments, in order to distinguish a single first bead with a codeable label from two beads with codeable labels that have a combined intensity similar to the intensity of the codeable label of the first bead, a single internal reference label in each bead may be included. In certain embodiments, detection of two internal reference labels would indicate the presence of two beads, while detection of a single internal reference label would indicate the presence of a single bead. Thus, in certain embodiments, internal reference labels assist in accurate determination of the number of beads actually present when detection of codeable labels alone may provide ambiguous results.

For example, in certain embodiments in which a bead comprises coding elements comprising fluorophores, dyes, or nanocrystals, the internal reference label may be a single quantum dot in each bead. The presence of a single quantum dot may be used to indicate the presence of a single bead. The presence of two quantum dots would indicate the presence of two beads, and so forth.

As another nonlimiting example, in certain embodiments, an internal reference label may provide a color signal that is detectably different from the signal of the codeable labels. In certain embodiments, the signal from the internal reference label for each bead will have an intensity that can be used to identify the presence of a single bead. For example, in certain embodiments, the internal reference signal for each bead will provide a red signal with an intensity of about one unit. In certain such embodiments, one may employ two different codeable labels on two different beads to detect two different targets. For example, in certain embodiments the first codeable label for a first target provides a green signal having an intensity of one unit, and the second codeable label for a second target provides a green signal having an intensity of two units. Without an internal reference label, in certain embodiments, one may have difficulty determining whether a green signal having an intensity of two units indicates the presence of two beads for the first target or the presence of one bead for the second target. In certain embodiments that employ the red internal reference label, the detection of a red signal with an intensity of one unit will indicate the presence of one bead for the second target, and the detection of a red signal with an intensity of two units will indicate the presence of two beads for the first target.

When beads of varying size are employed, the amount of label, such as a fluorescent dye as a non-limiting example, incorporated into such beads may vary according to the size of the bead. In certain embodiments, the inclusion of an internal reference label in beads may be used to normalize variations in codeable labels signal caused by variations in bead size.

In certain embodiments that do not employ an internal reference label, one tries to use beads of fairly uniform size to try to avoid differences in signal from the same codeable label due to the difference in the sizes of the beads. In certain embodiments, an internal reference label on the beads may permit one to use beads of varying size. In certain such embodiments, one may employ two different codeable labels on two different beads to detect two different targets. For example, if the beads have a diameter of X, the first codeable label for a first target provides a green signal having an intensity of one unit, and the second codeable label for a second target provides a green signal having an intensity of two units. Without an internal reference label, in certain embodiments with beads of varying size, one may have difficulty determining whether a bead providing a green signal having an intensity of two units indicates the presence of a bead for the first target having a diameter larger than X or the presence of a bead for the second target having a diameter X.

In certain such embodiments, one may employ beads that include an internal reference label that is detectably different from the codeable labels. In certain embodiments, one may employ an internal reference label that provides a red signal having an intensity of one unit if the bead has a diameter of X. Thus, if the bead size varies from the diameter of X, the internal reference label will provide a different intensity than one unit. In certain such embodiments, the detection of a bead with a green signal of two units indicates the presence of the second target if the red signal is one unit and indicates the presence of the first target if the red signal is two units.

In certain embodiments, the use of an internal reference label may allow one to produce beads of smaller sizes than is practical without the use of an internal reference label. In certain embodiments, beads may be less than 2µm in diameter. In certain embodiments, using an internal reference label, one may be able to distinguish very small differences in bead size.

In certain embodiments, the use of an internal reference label could be used when counting beads by staging or by flow cytometry. In certain embodiments, beads employing an internal reference label may be used in an array, wherein analytes are bound to specific regions of the array. In certain embodiments, arrays with beads with Internal reference labels may be imaged. In certain embodiments, software may be used to normalize signals using the internal reference labels in digitalized images.

In certain embodiments, the size of a bead may be used as a coding element. As a non-limiting example, beads have 100 different codes employing two colors. In certain embodiments, different sized beads may be used as part of the code, because different sized beads provide different intensities. For example, in certain embodiments, the 100 codes using two colors may be increased to 400 codes by using four different sized beads.

### Detectable Complexes

The term "detectable complex" of the present invention is a complex comprising codeable label. In certain embodiments, a detectable complex further comprises at least one probe.

According certain embodiments, a detectable complex is produced when a target is present and is not produced when a target Is absent. In certain embodiments, a detectable complex is formed if the target and probe specifically bind one another.

In certain embodiments, the detectable complex is produced in a ligation reaction. Ligation methods include, but are not limited to, both enzymatic and chemical ligation.

A ligation reaction according to the present invention comprises any enzymatic or chemical process wherein an internucleotide linkage is formed between the opposing ends of nucleic acid sequences that are adjacently hybridized to a template. Additionally, the opposing ends of the annealed nucleic acid sequences typically are suitable for ligation (suitability for ligation is a function of the ligation method employed). The internucleotide linkage may include, but is not limited to, phosphodiester bond formation. Such bond formation may include, without limitation, those created enzymatically by a DNA or RNA ligase, such as bacteriophage T4 DNA ligase, T4 RNA ligase, *Thermus thermophilus* (*Tth*) ligase, *Thermus aquaticus* (*Taq*) ligase, or *Pyrococcus furiosus* (*Pfu*) ligase. Other internucleotide linkages include, without limitation, covalent bond formation between appropriate reactive groups such as between an α-haloacyl group and a phosphothioate group to form a thiophosphorylacetylamino group, a phosphorothioate a tosylate or iodide group to form a 5'-phosphorothioester, and pyrophosphate linkages.

Chemical ligation agents include, without limitation, activating, condensing, and reducing agents, such as carbodiimide, cyanogen bromide (BrCN), N-cyanoimidazole, imidazole, 1-methylimidazole/carbodiimide/ cystamine, dithiothreitol (DTT) and ultraviolet light. Autoligation, i.e., spontaneous ligation in the absence of a ligating agent, is also within the scope of the invention. Detailed protocols for chemical ligation methods and descriptions of appropriate reactive groups can be found, among other places, in Xu et al., Nucleic Acid Res., 27:875-81 (1999); Gryaznov and Letsinger, Nucleic Acid Res. 21:1403-08 (1993); Gryaznov et al., Nucleic Acid Res. 22:2366-69 (1994); Kanaya and Yanagawa, Biochemistry 25:7423-30 (1986); Luebke and Dervan, Nucleic Acids Res. 20:3005-09 (1992); Sievers and von Kiedrowski, Nature 369:221-24 (1994); Liu and Taylor, Nucleic Acids Res. 26:3300-04 (1999); Wang and Kool, Nucleic Acids Res. 22:2326-33 (1994); Purmal et al., Nucleic Acids Res. 20:3713-19 (1992); Ashley and Kushlan, Biochemistry 30:2927-33 (1991); Chu and Orgel, Nucleic Acids Res. 16:3671-91 (1988); Sokolova et al., FEBS Letters 232:153-55 (1988); Naylor and Gilham, Biochemistry 5:2722-28 (1966); and U.S. Patent No. 5,476,930.

In certain embodiments, one may employ at least one cycle of the following sequential procedures: hybridizing the sequence-specific portions of a first target-specific probe and a second target-specific probe, that are suitable for ligation, to their respective complementary target regions; ligating the 3' end of the first target-specific probe with the 5' end of the second target-specific probe to form a ligation product; and denaturing the nucleic acid duplex to separate the ligation product from the target sequence. The cycle may or may not be repeated. For example, without limitation, by thermocycling the ligation reaction to linearly increase the amount of ligation product.

Also within the scope of the invention are ligation techniques such as gap-filling ligation, including, without limitation, gap-filling OLA and LCR, bridging oligonucleotide ligation, and correction ligation. Descriptions of these techniques can be found, among other places, in U.S. Patent Number 5,185,243, published European Patent Applications EP 320308 and EP 439182, and published PCT Patent Application WO 90/01069.

Detectable complexes may also be produced by hybridization of nucleic acids without any ligation steps. In certain embodiments, hybridization occurs with PNA, LNA, or other synthetic nucleic acids that have a higher Tm than naturally occurring nucleic acid hybridizations.

Other detectable complexes also may be produced by antibody-antigen interactions, aptomer-protein interactions, and action of other specific binding pairs (e.g., streptavidin- biotin reactions).

Detectable complexes may also be produced by primer extension reactions. Primer extension reactions include, but are not limited to, single base extension (SBE) reactions, sequencing reactions (for example Sanger dideoxy sequencing reactions), and other reactions including polymerase.

In certain embodiments, the detectable complex is produced in a ligand-receptor reaction. As a non-limiting example, a codeable label and a probe may be attached to the ligand molecule. The receptor is attached to a separating moiety, such as a magnetic bead.

In certain embodiments, a probe is hybridized to a target nucleic acid sequence, and a codeable label bound to a single nucleotide is attached to the probe by a polymerase reaction when the target nucleic acid is present.

In certain embodiments, a probe comprising a nucleic acid, complementary to a nucleic acid target sequence, is attached to a separating moiety such as a magnetic bead. In certain embodiments, the probe is then added to a sample containing the nucleic acid target sequence. In certain embodiments, codeable labels attached to nucleotides are added to the sample with a polymerase. In certain embodiments, if the nucleic acid target sequence is present in the sample, the probe hybridizes to the target, and the polymerase adds the codeable label-attached nucleotides to the oligonucleotide probe, forming a detectable complex. In certain embodiments, after denaturation from the sample nucleic acids, the probes are then separated from the sample using the magnetic beads. In certain embodiments, if a detectable complex is counted, then a target is present in the sample.

In certain embodiments, the number of targets in a sample is represented by the number of detectable complexes removed from a sample as compared to the number of detectable complexes present at the start of a detection reaction. In certain embodiments, a number of detectable complexes are added to a sample. In certain embodiments, these detectable complexes comprise a codeable label attached to one end of a single-stranded nucleic acid probe, and a separating moiety attached to the other end of the single-stranded nucleic acid probe. In certain embodiments, when a target is present in the sample, the target hybridizes to the single-stranded probe to form a double stranded molecule. In certain embodiments, an endonuclease is added to the reaction which cuts double-stranded nucleic acid. In such embodiments, the number of detectable complexes remaining is equal to the number of detectable complexes initially added to the reaction less the number of targets present in the sample.

### Separating Moieties and Methods

The term "separating moiety" refers to any moiety that, when included in a detectable complex, may be used to separate the detectable complex from at least one other moiety in the sample.

In certain embodiments, separation is achieved without any particular separating moiety incorporated in a detectable complex. In certain embodiments, methods that do not employ a specific separating moiety include, but are not limited to, separation based on density, size, electrical or ionic charge, diffusion, heat, flow cytometry, and directed light. In certain embodiments, the detection of detectable complexes occurs without any separation of detectable complexes from other moieties.

In certain embodiments, the methods comprise separating the detectable complex from separating moieties that are not in a detectable complex, prior to the quantitating, or the detecting the presence or absence of, one or more targets. One of ordinary skill will appreciate that there are several methods that may be used according to certain embodiments for separating detectable complexes from separating moieties not in a detectable complex. As non-limiting examples in certain embodiments, differences in density or size of separating moieties may be used to separate detectable complexes from separating moieties not in a detectable complex. Methods of separation include, but are not limited to, use of sizing filters, sizing columns, density gradients, separation by gravity, and separation by centrifugation. Examples of such size-separating moieties include, but are not limited to, polymer beads.

For example, in certain embodiments, one may separate detectable complexes from separating moieties not in a detectable complex as follows. Probe sets may include a separating bead comprising a first probe and a detecting bead comprising a second probe. The separating bead further comprises a first codeable label, and the detecting bead further comprises a second codeable label. The separating beads are smaller in size than the detecting beads. After ligation, one can separate detectable complexes from separating beads based on the differences in sizes of the detecting beads in the detectable complexes and the separating beads not in detectable complexes. For example, one may pass the material through a sizing filter that allows separating beads to flow through and which retains detectable complexes.

Also, in certain embodiments, one may separate detectable complexes from separating moieties not in detectable complexes as follows. Probe sets may include a separating bead comprising a first probe and a detecting bead comprising a second probe. The separating bead further comprises a first codeable label, and the detecting bead further comprises a second codeable label. The separating beads have a higher density than the detecting beads. After a detectable complex is formed, one can separate detectable complexes from separating beads not in a detectable complex, based on the differences in density of the detecting beads in the detectable complexes and the free separating beads. For example, in certain embodiments, one may place the material in a density gradient, which will separate the detectable complexes from the free separating beads. Also, in certain embodiments, gravity may be used to separate the detectable complexes from the free separating beads. Thus, if the separating beads have a higher density than the detecting beads, the separating beads will sink below the detectable complexes.

According to certain embodiments, other different properties of the probes in a probe set may be used to separate detectable complexes from probes and codeable labels not in detectable complexes. For example, in certain embodiments, one may use a separating moiety that has a particular property that attracts it to a particular position and other moieties in the reaction mixture that lack that property. For example, according to certain embodiments, the separating moiety may comprise a magnetic particle and the other moieties in the reaction mixture do not comprise a magnetic particle.

The term "magnetic particle" refers to material which can be moved using a magnetic force. This includes, but is not limited to, particles that are magnetized, particles that are not magnetized but are influenced by magnetic fields (e.g., colloidal iron, iron oxides (e.g., ferrite and magnetite), nickel, and nickel-iron alloys), and particles which can become magnetized (e.g., ferrite, magnetite, iron, nickel, and alloys thereof).

In certain embodiments, the magnetic particle comprise one or more of ferrite, magnetite, nickel, and iron, and the other moieties in the reaction mixture do not comprise such a material. In such embodiments, one can use such distinctive properties of the separating moiety to separate detectable complexes that include a separating bead from probes and codeable labels not in detectable complexes.

Other methods of separating detectable complexes from other moieties include, but are not limited to, separation by density, separation by electrical charge, separation by drag coefficiencies (e.g., electrophoretic mobility), separation by diffusion or dialysis, and separation by heat or light (e.g., employing lasers to move labeled particles).

In certain embodiments, one may remove separating moieties, codeable labels, or probes not in detectable complexes (free components) from a composition containing detectable complexes prior to the quantitating the target nucleic acid sequence or sequences in the sample. In certain embodiments, one may remove detectable complexes from a composition containing free components prior to the quantitating (or detecting the presence or absence of) the target nucleic sequence or sequences in the sample.

In certain embodiments, separating the detectable complex from free components comprises separating the detectable complex from the target nucleic acid sequence, and separating the detectable complex from the sample.

In certain embodiments, the detectable complex is a ligation product. In certain embodiments, separating of the detectable complex from the target sequence comprises thermal denaturation.

As a nonlimiting illustration, in certain embodiments, one may detect the presence or absence of different target nucleic acid sequences in a sample, such as a cell lysate, as follows. A sample is combined with a different probe set specific for each of the different target nucleic acid sequences. Each probe set comprises a separating bead comprising a magnetic particle incorporated into a bead and a first target-specific probe and comprises a detecting bead comprising a bead and a second target-specific probe. The separating beads of the probe sets have a higher density than the detecting beads.

The separating bead of each probe set further comprises a first codeable label that is specific for the first target-specific probe, and the detecting bead of each bead set further comprises a second codeable label that is specific for the second target-specific probe. The first codeable label is detectably different from the second codeable label. The target-specific probes in each bead set are suitable for ligation together when hybridized adjacent to one another on a complementary target sequence.

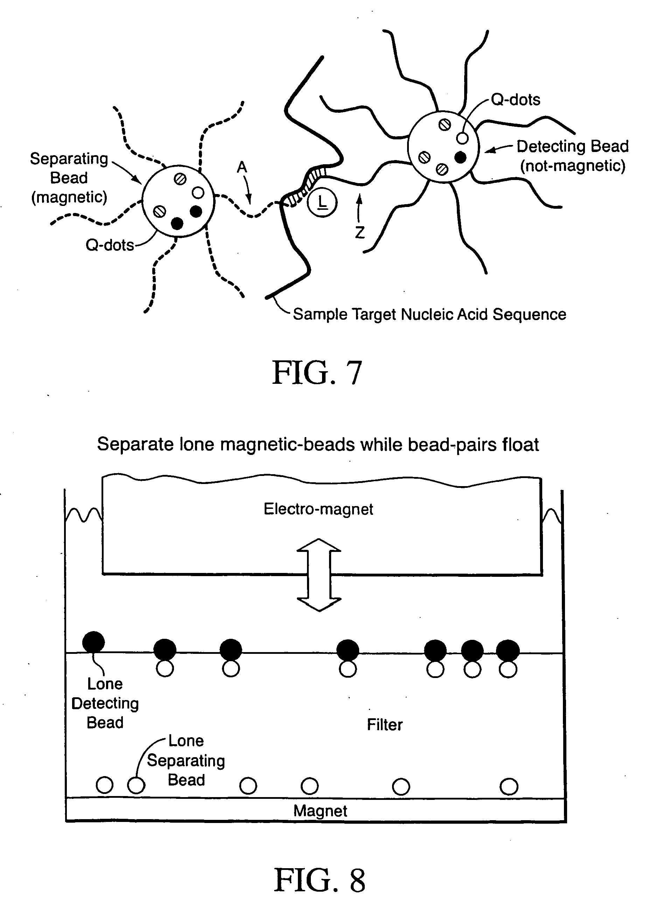

When the sample includes a complementary target nucleic acid sequence to the first and second target-specific probes of a given probe set, the probes anneal and are ligated in the presence of ligase (L) to form a detectable complex comprising the separating bead and the detecting bead (see, e.g., Figure 7). After ligation, the target nucleic acid sequence is thermally denatured from the detectable complex.

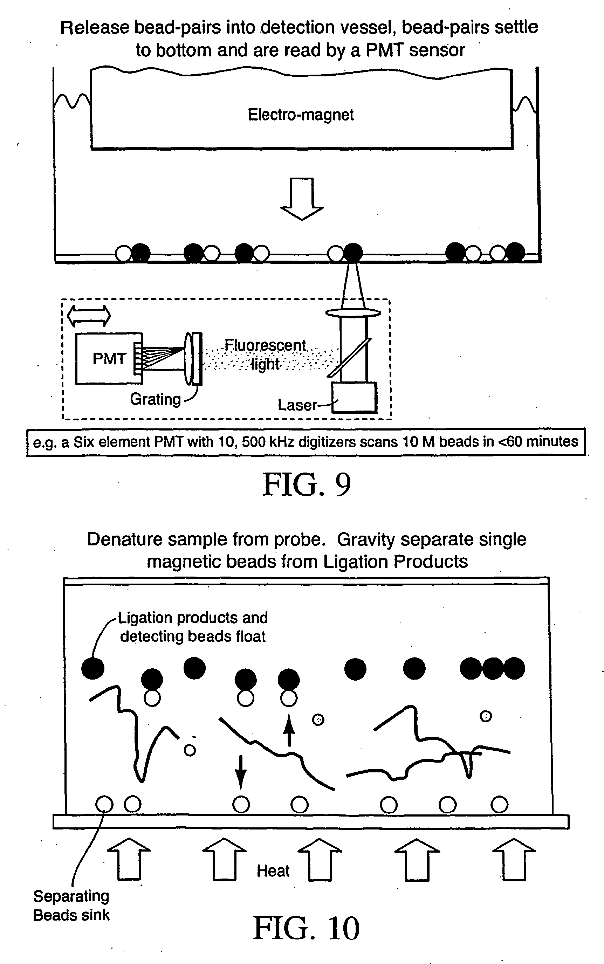

In certain embodiments depicted in Figures 8 and 9, the sample is then subjected to a density gradient such that detectable complexes and detecting beads are situated above separating beads in the vessel. Detectable complexes may then be separated from unligated detecting beads by a magnetic source (see Figure 8). For example, one can remove the detectable complexes from the sample containing the unligated detecting beads using the magnetic source, and can place the detectable complexes in a separate vessel that does not contain any unligated beads (see Figures 8 and 9). One can then detect the presence or absence of detectable complexes by counting of the unique combinations of codeable labels.

In certain embodiments, one may use a second magnet near the bottom of the vessel, which will attract and hold the higher density unligated separating beads but will not attract or hold the lower density detectable complexes and unligated detecting beads (see Figure 8). Such a second magnet near the bottom of the vessel, however, is not mandatory. For example, in certain embodiments, one can design the density of the beads such that distance of separation between any unligated separating beads and the detectable complex allows attraction of the detectable complex to a magnetic device and does not allow attraction of the unligated separating beads to the magnetic device.

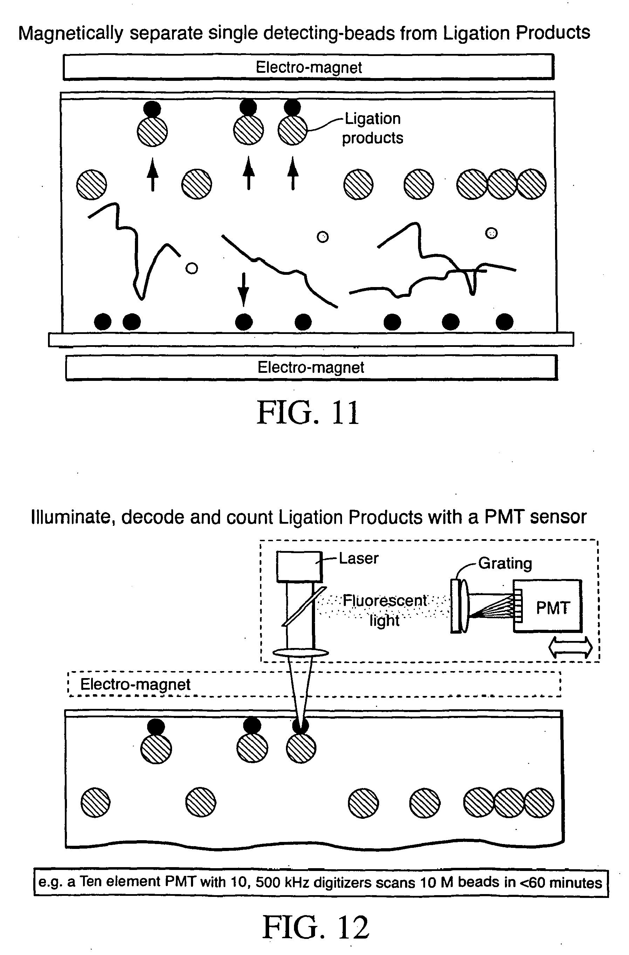

In certain embodiments depicted in Figures 10 and 11, after ligation, the sample is heated to denature the hybridized probes and target nucleic acid sequences. Due to gravity, the unligated separating beads sink below the detectable complexes and unligated detecting beads (see Figure 10). One may then place an electro-magnet (magnet) near the bottom of the vessel such that it attracts and holds the higher density unligated separating beads, and such that it does not attract or hold the lower density detectable complexes and unligated detecting beads (see Figure 11). Also, one may place an electro-magnet (magnet) into the top of the vessel such that it attracts and holds detectable complexes (see Figure 11).

The electro-magnet holding the detectable complexes may then be lifted to separate the detectable complexes from the unligated detecting beads (see Figures 11 and 12). One can then detect the presence or absence of detectable complexes, without removing them from the sample including the unligated beads, by counting the unique combinations of codeable labels (see Figure 12). Figure 12 depicts certain embodiments where detectable complexes are illuminated, the codes are identified, and ligation products are counted with a PMT sensor.

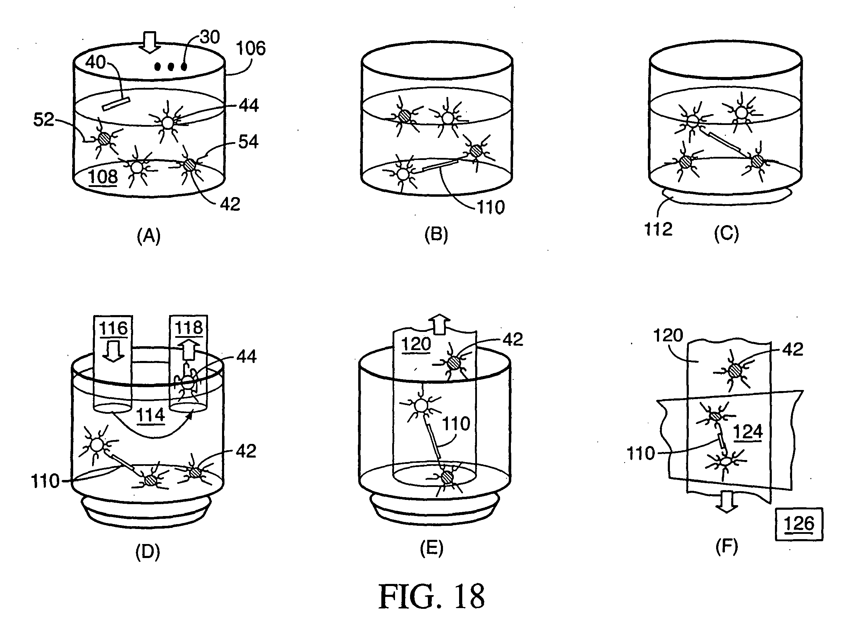

In certain embodiments depicted in Figure 18, detectable complexes are formed comprising a magnetic bead comprising a first codeable label and a nonmagnetic bead comprising a second codeable label. In certain embodiments, an electromagnet (magnet) is placed beneath a reaction vessel containing beads and detectable complexes. When the electromagnet is turned on, detectable complexes and magnetic beads that are not in a detectable complex are attracted to the bottom of the vessel. See Figure 18 C.

In certain embodiments, nonmagnetic beads that are not in a detectable complex and other nonmagnetic moieties are removed by a continuous flow system, comprising an input tube and an output tube. See Figure 18D. In certain embodiments, the electromagnet is then turned off, and the detectable complexes and the magnetic beads that are not in a detectable complex are then pulled out with a flow cytometer tube. See Figure 18E.

In certain embodiments, the detectable complexes and the magnetic beads that are not in a detectable complex are then sent through a flow cytometer and only combinations of the first and second codeable labels are counted. See Figure 18F. In such embodiments, the magnetic beads that are not in a detectable complex will include only a first codeable label, which will not be counted.

In certain embodiments, one may carry out the method discussed above for Figure 18 with a magnetic bead that does not include a codeable label. After separation of the nonmagnetic beads that are not in a detectable complex, the detectable complexes and the magnetic beads that are not in a detectable complex are then sent through a flow cytometer. Since the magnetic beads do not have codeable label in such embodiments, only the codeable labels of the nonmagnetic beads in the detectable complexes are counted.

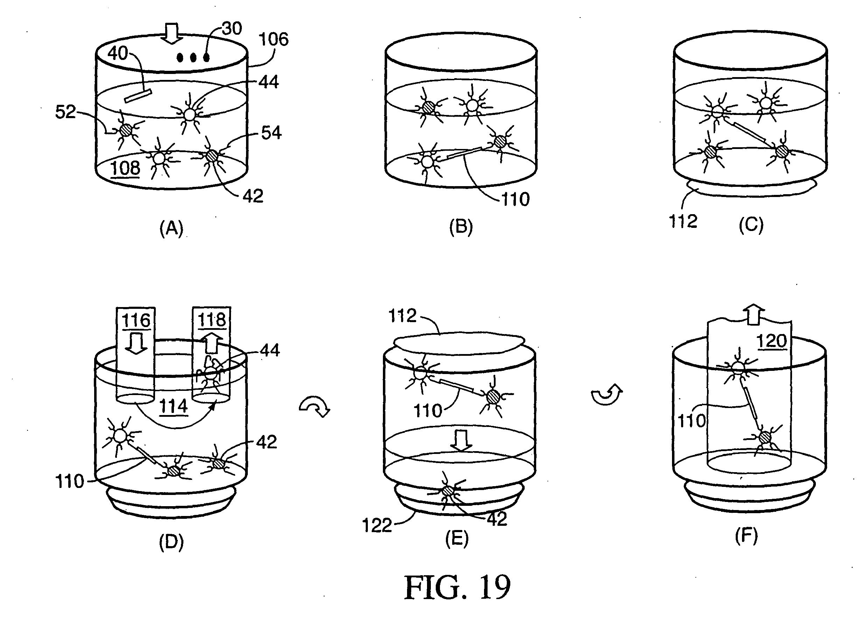

In certain embodiments depicted in Figure 19, detectable complexes are formed comprising a magnetic bead, a nonmagnetic bead, and a codeable label. In certain embodiments, a first electromagnet is placed beneath a reaction vessel containing beads and detectable complexes. When the first electromagnet is turned on, detectable complexes and magnetic beads that are not in a detectable complex are attracted to the bottom of the vessel. See Figure 19 C.

In certain embodiments, nonmagnetic beads that are not in a detectable complex and other nonmagnetic moieties are removed by a continuous flow system, comprising an input tube and an output tube. See Figure 19D. In certain embodiments, a vessel is used that may be inverted such that a substantial amount of liquid will not drain out when it is inverted. In certain embodiments, this may be accomplished using a small_vessel in which surface tension inhibits drainage of liquid out of the vessel when the vessel is inverted. In embodiments that employ an inverted vessel, the vessel and the first electromagnet are then inverted and the first electromagnet is turned off. A second electromagnet is then turned on at the bottom of the inverted vessel to attract the detectable complexes and the magnetic beads that are not in a detectable complex. See Figure 19E. In certain embodiments, the detectable complexes have more drag and less density than the magnetic beads that are not in a detectable complex. Thus, in such embodiments, the magnetic beads that are not in a detectable complex move faster than the detectable complexes toward the second electomagnet. After the magnetic beads that are not in a detectable complex are collected onto the second electromagnet (see Figure 19E), the vessel is inverted back before the detectable complexes have reached the second electromagnet.

In certain embodiments, the detectable complexes are then pulled out with a flow cytometer tube (see Figure 19F), and are sent through a flow cytometer and the codeable labels of the detectable complexes are counted.

In certain embodiments, one may carry out the method discussed above for Figure 19 with a magnetic bead that does not include a codeable label. In certain embodiments, one may carry out the method discussed above for Figure 19 with a nonmagnetic bead that does not include a codeable label.

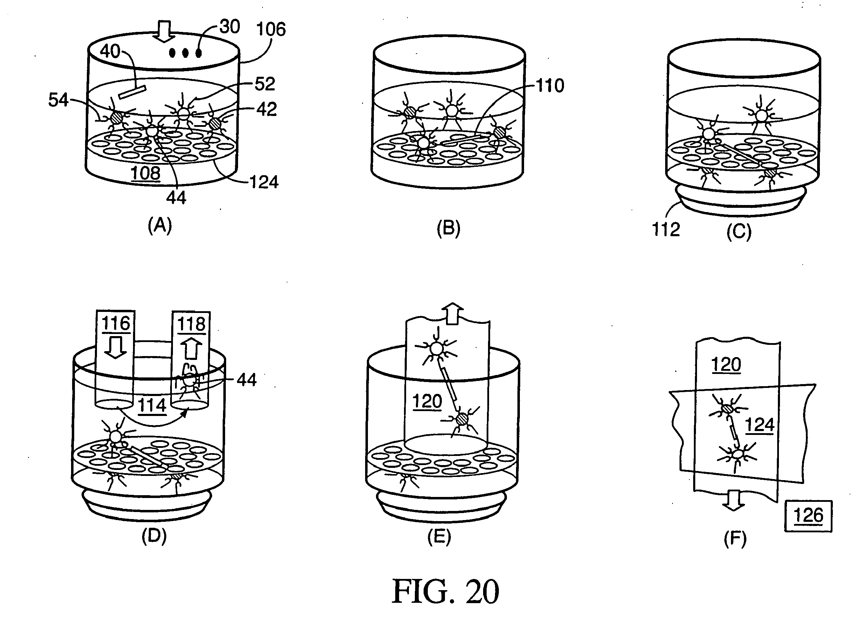

In certain embodiments depicted in Figure 20, detectable complexes are formed comprising a magnetic bead, a nonmagnetic bead, and a codeable label. A filter is included in the vessel. In certain embodiments, the magnetic beads are designed such they can pass through the filter and the nonmagnetic beads are designed such that they cannot pass through the filter. In certain embodiments, an electromagnet is placed beneath the reaction vessel containing beads and detectable complexes. When the first electromagnet is turned on, detectable complexes and magnetic beads that are not in a detectable complex are attracted to the bottom of the vessel. See Figure 20 C.

The magnetic beads that are not in a detectable complex pass through the filter toward the magnet. The detectable complexes are pulled toward the magnet, but cannot pass through the filter in view of the nonmagnetic bead of the complex. The detectable complexes are held at the filter by the pull of the magnet. In certain embodiments, nonmagnetic beads that are not in a detectable complex and other nonmagnetic moieties are then removed by a continuous flow system, comprising an input tube and an output tube. See Figure 20D. In certain embodiments, detectable complexes can then be separated from magnetic beads that are not in a detectable complex by moving the filter away from the electromagnet. Such movement of the filter pulls the detectable complexes away from the electromagnet and away from the magnetic beads that are not in a detectable complex.

In certain embodiments, detectable complexes are then pulled out with a flow cytometer tube. See Figure 20E. In certain embodiments, the detectable complexes are then sent through a flow cytometer and the codeable labels of the detectable complexes are counted. See Figure 20 F.

In certain embodiments, one may carry out the method discussed above for Figure 20 with a magnetic bead that does not include a codeable label. In certain embodiments, one may carry out the method discussed above for Figure 20 with a nonmagnetic bead that does not include a codeable label.

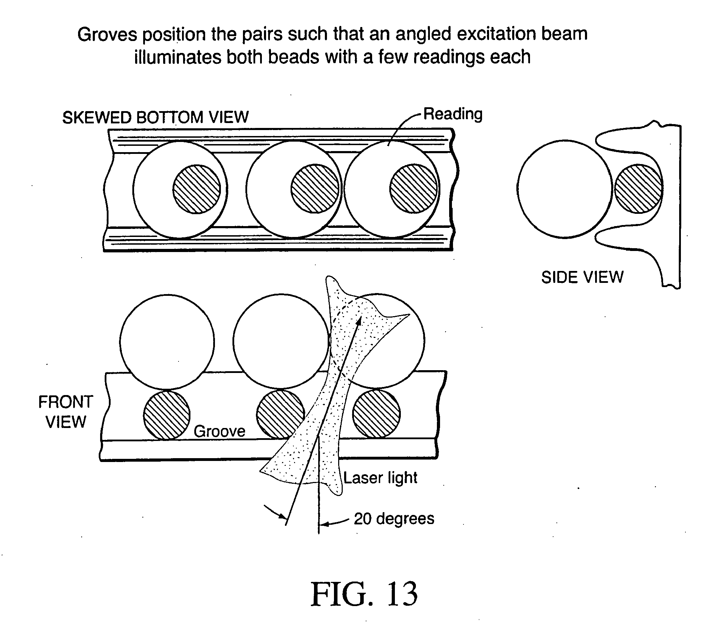

In certain embodiments, one may use grooves in a vessel that help to align detectable complexes in a manner that facilitates the detection of the presence or absence of sets of labels. In certain embodiments, "aligned ligation products" are products in which the separating beads of the products are closer to a given surface of a vessel than the detecting beads. For example, in certain embodiments depicted in Figure 13, the separating beads may be smaller in size than the detecting beads. A groove is designed such that the separating beads fit into the groove, and the separating beads are too large to fit into the groove (see Figure 13). In certain embodiments, one may place a magnetic source near the groove in the vessel to attract and hold separating beads into the groove. One can then count the combinations of codeable labels to detect the presence or absence of detectable complexes. In certain embodiments shown in Figure 13, the grooves position the detectable complexes such that an angled excitation beam illuminates both beads with a few readings.

In certain embodiments, electrophoresis may also be used to separate separating moieties by charge or by a charge:mass ratio. In certain embodiments, a charged separating moiety may also be separated by ion exchange, e.g., by using an ion exchange column or a charge-based chromatography.

In certain embodiments, a separating moiety may also be a member of an affinity set. An affinity set is a set of molecules that specifically bind to one another. Exemplary affinity sets include, but are not limited to, strepavidin-biotin pairs, complementary nucleic acids, antibody-antigen pairs, and affinity binding chemicals available from Prolinx^{™} (Bothell, WA) as exemplified by U.S. Patent Nos. 5,831, 046; 5,852,178; 5,859,210; 5,872,224; 5,877,297; 6,008,406; 6,013,783; 6,031,17; and 6,075,126.

In certain embodiments, separating moieties are separated in view of their mobility. In certain embodiments, separating in view of mobility is accomplished by the size of the separating moiety. In certain embodiments, mobility modifiers may be employed during electrophoresis. Exemplary mobility modifiers and methods of their use have been described, e.g., in U.S. Patent Nos. 5,470,705; 5,580,732; 5,624,800; and 5,989,871. In certain embodiments, by changing the mobility of a codeable label, one may distinguish signals associated with the presence of a target from signals from labels not associated with the presence of a target.

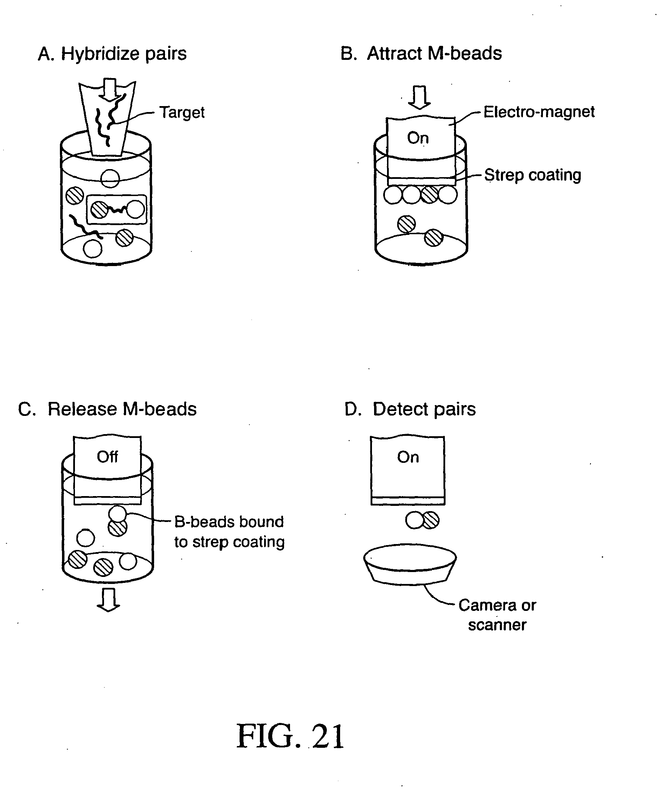

In certain embodiments, two or more different separating moieties or methods may be used. As a nonlimiting example, in certain embodiments, a detectable complex may comprise a magnetic bead, a ligation product, and a biotin-coated bead. See, e.g., Figure 21, part A. A streptavidin-coated electromagnet is placed in the sample and turned on See, e.g., Figure 21, part B. The detectable complexes and the magnetic beads that are not in a detectable complex are attracted to the electromagnet. The biotin-coated beads in the detectable complexes bind to the streptavidin on the electromagnet. See, e.g., Figure 21, part C. The electromagnet is then turned off, and the magnetic beads that are not in a detectable complex fall off the electromagnet, while the detectable complexes remain bound to the electromagnet. In certain embodiments, the electromagnet is then removed from the sample with the detectable complexes bound to the electromagnet, and the codeable labels in the detectable complexes are detected by camera or scanner. See, e.g., Figure 21, part D.

In certain embodiments, the presence of a target prevents, rather than facilitates, the formation of a detectable complex. In certain such embodiments, the number of probes and/or codeable labels are limited. In certain such embodiments, counting of detectable complexes provides a number of codeable labels that are not associated with targets. Subtracting the number of detectable complexes that are counted from the total number of detectable complexes expected in the complete absence of targets provides the number of targets present in the sample.

### Tube in Tube Separation of Detectable Complexes

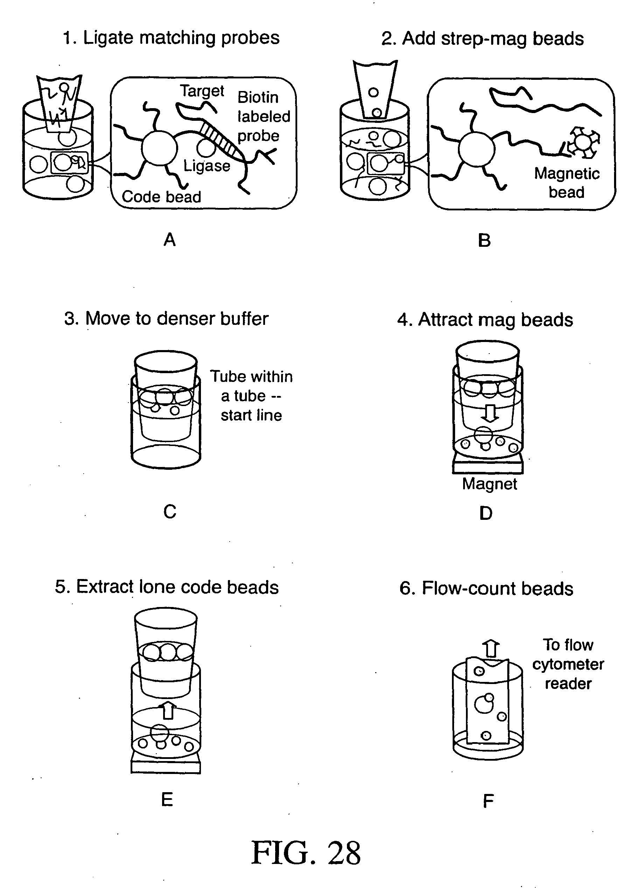

In certain embodiments depicted in Figure 28, detectable complexes are formed comprising a bead comprising a codeable label and a separating moiety, such as a biotin molecule. In certain embodiments, one may include a ligation reaction in the process of forming detectable complexes. In certain embodiments, one may include an antibody-peptide reaction in the process of forming detectable complexes. In certain embodiments, separating moieties other than biotin may be employed.

In the embodiments depicted in Figure 28, a probe set is employed that includes a first probe with a bead comprising a codeable label and a second probe attached to biotin. In Figure 28 A, ligation occurs if the target is present to form the detectable complex. In certain embodiments shown in Figure 28(B), streptavidin-coated magnetic beads are added. The streptavidin-coated magnetic beads bind to biotin molecules in the detectable complexes. In certain embodiments, the beads comprising codeable labels that are not ligated to biotin molecules do not bind to the streptavidin-coated magnetic beads. In certain embodiments, the process shown in Figure 28 may be modified by employing a process that does not involve ligation to form a detectable complex comprising a bead comprising a codeable label and biotin.

As shown in certain embodiments depicted in Figure 28(C), a first tube is provided that is placed within a second tube which is larger. In certain embodiments, the two tubes are partially filled with a buffer that has a density greater than the beads comprising a codeable label. In certain embodiments, the second tube is partially filled with the buffer, and the second tube is placed in the outer tube, such that the buffer comes to an equilibrium height in the first and second tubes.

In certain embodiments, after the ligation reaction, at least a portion of the sample is placed on top of the high density buffer inside the first tube, such that the beads float in the buffer. In certain embodiments depicted in Figure 28(D), a magnet is then applied to the bottom of the second tube such that unbound streptavidin-coated magnetic beads and detectable complexes are attracted to the magnet at the bottom of the second tube. Beads comprising codeable labels not in a detectable complex remain floating on or toward the top of the high density buffer within the first tube. In certain embodiments depicted in Figure 28(E), the top of the first tube is substantially sealed sealed, and the first tube is lifted above the buffer in the second tube. Thus, the beads comprising codeable labels not in detectable complexes are separated from the buffer containing the detectable complexes. In certain embodiments, the first tube prevents beads from sticking to the walls of the second tube.

In certain embodiments, the detectable complexes in the second tube may then be removed to be counted. In certain embodiments, the detectable complexes are subjected to a using a flow cytometer. In certain embodiments, the magnet on the bottom of the second tube is removed with the detectable complexes, and the codeable labels are detected on the magnet.

### Detection Methods

In certain embodiments, the present invention provides for the detection of codeable labels. In certain embodiments, the present invention provides for the counting of labels. Several methods of label detection and/or counting are envisioned, and one of skill in the art will appreciate the variety of methods by which one could detect and/or count codeable labels of the present invention.

As discussed above, counting of codeable labels refers to the actual counting of individual labels. In certain embodiments, detection and/or counting further includes identifying the code of a label if multiple detectably different labels are employed in the same procedure.

In certain embodiments, codeable labels are detected with a type of flow cytometry, such as a Fluorescence Associated Cell Sorter (FACS), a Luminex^{™} detection device, or a similar technology developed for the detection of single codeable label molecules. In certain embodiments, codeable labels are resolved by electrophoresis and detected during or after electrophoretic migration of the codeable labels. Electrophoresis includes, but is not limited to, capillary electrophoresis and field electrophoresis. In certain embodiments, such methods involve a device that excites the codeable labels (such as a laser, as a non-limiting example) and a scanning device that counts the codeable labels. In certain embodiments shown in Figure 9, detectable complexes are released into a detection vessel and detectable complexes that settle to the bottom of the vessel are read by a PMT sensor. In certain embodiments, the PMT sensor is a six element PMT device with ten 500 kHz digitizers that scan 10 million beads in under 60 minutes.

In certain embodiments, other methods of detection involve static methods of detection. In certain embodiments, such methods involve placing the codeable labels or complexes on a plate (as a non-limiting example), exciting the codeable labels with one or more excitation sources (such as lasers or different wavelengths, for example) and running a scanning device across the plate in order to count the codeable labels. In certain embodiments, the plate is moved back and forth across the field of detection of the scanning device. In certain embodiments, the codeable labels are attached to the plate or slide. In certain embodiments, a camera could image the entire field, and the image could be scanned in order to count the codeable labels.

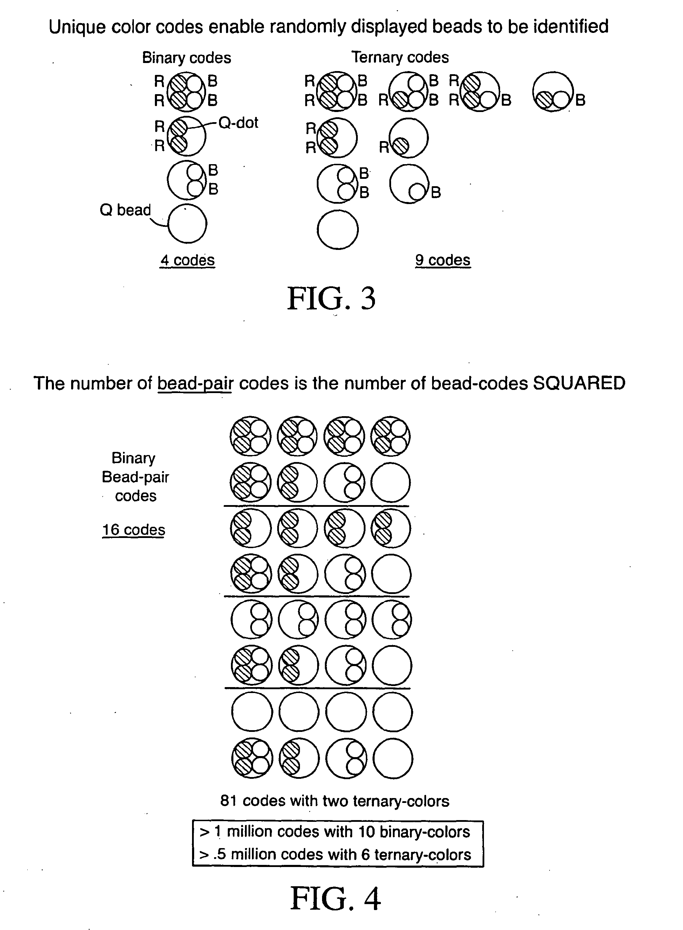

According to certain embodiments, multiple targets may be detected in a sample, and distinguished by using different codeable labels. In certain embodiments, the codeable labels can be coded using two or more labels (e.g., in certain embodiments, quantum dots, fluorophores, or dyes are used). In certain embodiments, one may use multiple wavelengths or colors of labels, which multiplies the number of potential different codeable labels. For example, if a given codeable label is given a binary code, then one can detect the presence or absence of a specific color of label (either a "1" or "0" - hence a binary code). If only one binary color is used, then there are 2 codes, one with the label, and one without the label. If two binary colors are used (e.g., red and blue), then 4 codes are possible - (1) red, (2) blue, (3) red and blue, and (4) no color (see, e.g., Fig. 3). Each additional color multiplies the number of possible codes by two. Thus, if 10 colors of labels are used, 1,024 binary codes are possible.

In certain embodiments, the codeable labels are incorporated or attached to beads. In certain embodiments, the codeable labels may be attached directly to probes without being incorporated into beads.

Intensity may also be used as a factor in distinguishing codeable labels. In certain embodiments, intensity variations may be accomplished using codeable labels that include the same number of labels of a single wavelength, but different codeable labels with different probes have labels with different intensity levels. In certain embodiments, intensity variations may be accomplished using codeable labels that include the same number of labels of a single emission spectrum, but different codeable labels with different probes have labels with different intensity levels. In certain embodiments, intensity variations may be accomplished by varying the number of labels of the same wavelength in different codeable labels attached to different probes. In certain embodiments, intensity variations may be accomplished by varying the number of labels of the same emission spectrum in different codeable labels attached to different probes. For example, in certain embodiments, one can use labels of the same wavelength in different codeable labels, and distinguish between the codeable labels using different numbers of labels in each different set. For example, if a codeable label is given a ternary code (three levels of intensity for each color of label), then one color of label provides three possible codes - (1) no label, (2) one label, and (3) two labels. If two colors are used, then 9 ternary codes are possible (see the nonlimiting example in Figure 3). Six colors would allow 729 ternary codes.

Further, when codeable labels are attached to two probes of a probe set (for example, by incorporation into beads), the number of potential codes is further multiplied (see the non-limiting examples in Figure 4). For example, using two colors in a binary code, 16 different probe set codes are possible (4 X 4). Using two colors in a ternary code, 81 different probe set codes are possible (9 X 9). Using 10 colors in a binary code, over 1 million probe set codes are possible (1,024 X 1,024). Using 6 colors in a ternary code, over 500,000 probe set codes are possible (729 X 729).

In addition, in certain embodiments, the labels, such as quantum dots for example, are particularly efficient in transmitting a signal such that codeable label can be detected. In certain such embodiments, the codeable labels may be used to detect very few molecules within a sample without target amplification.

In certain embodiments, a probe set comprises a separating bead that comprises a separating moiety, a first probe, and a first codeable label; and comprises a detecting bead that comprises a second probe and a second codeable label. In certain such embodiments, the first codeable label has a level of intensity that is specific for the first probe. In certain such embodiments, the second codeable label has a level of intensity that is specific for the second probe. In certain embodiments, the beads of a probe set comprise labels of the same wavelength, but the first codeable label has a level of intensity that is specific for the first probe, and the second codeable label has a level of intensity that is specific for the second probe.

In certain embodiments, the codeable label comprises at least 1,000 labels, wherein the labels have predetermined wavelength combinations that make each codeable label distinguishable from other codeable labels.

In certain embodiments, the codeable labels may comprise any number of labels from two to over 1,000. In certain embodiments, one uses codeable labels that allow one to detect the presence or absence of particular target-specific probes in a detectable complex. In certain embodiments, one uses codeable labels such that the detection of the presence of a particular combination of labels confirms the presence of one specific detectable complex. And, the detection of the absence of such a particular combination of labels confirms the absence of that one specific detectable complex.

In certain embodiments, the labels are selected from quantum dots, phosphors, and fluorescent dyes.

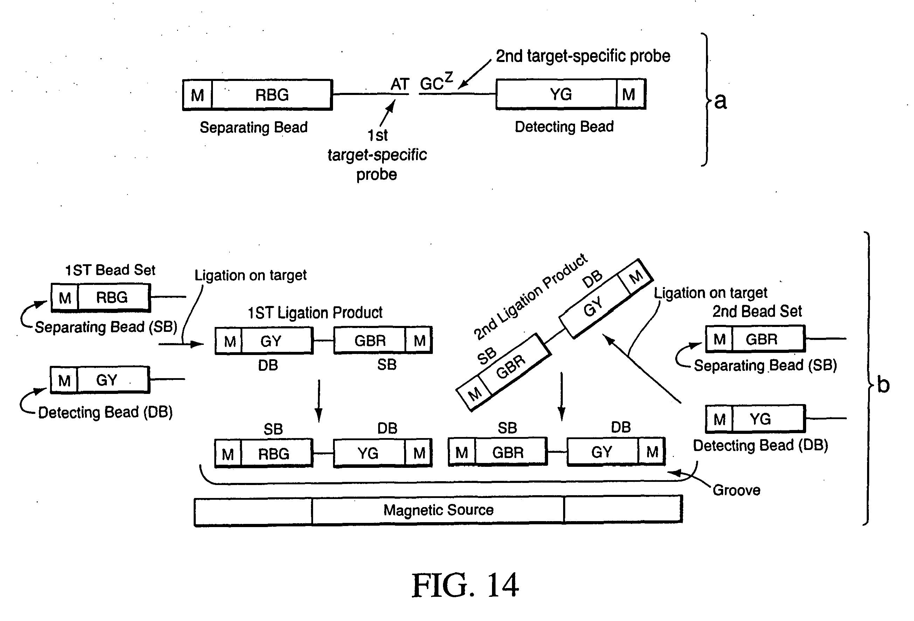

In certain embodiments that employ a first bead and a second bead, both the separating bead and detecting bead of the probe sets comprise a magnetic particle. In certain such embodiments, the beads are elongated and comprise a magnetic particle on one end and a target-specific probe on the other end. The beads further comprise labels placed in a particular order along the length of the bead (see, e.g., Figure 14(a)). See, e.g., U.S. Patent No. 4,053,433, which describes elongated polymers with labels in particular orders.

In certain embodiments, the polarity or orientation of the magnetic particles in the beads is designed to facilitate alignment of the detectable complexes. For example, in certain embodiments, the vessel containing the detectable complexes will include a groove on a surface that is placed near a magnetic source (see, e.g., Figure 14(b)). The beads are designed so that the polarity or orientation of the magnetic particles in the beads results in the detectable complexes aligning in the groove with the first bead of each detectable complex closer to one end of the groove than the second bead of that detectable complex (see, e.g., Figure 14(b)). One can then quantify detectable complexes by quantitating the particular order of combinations of codeable labels.

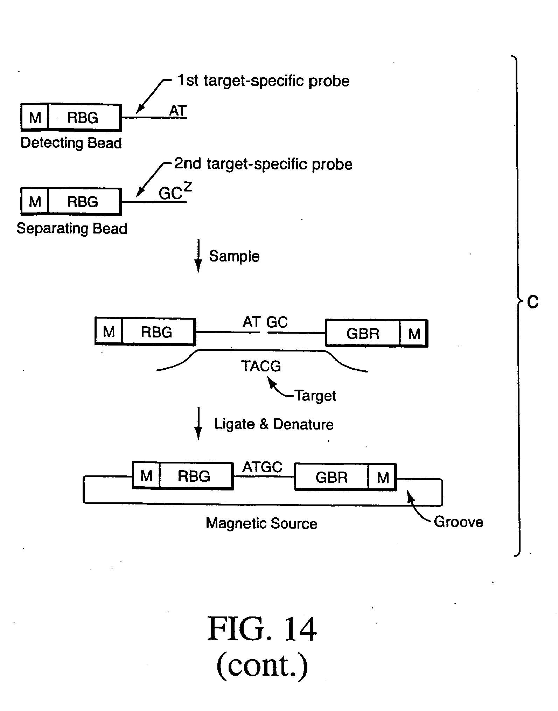

In such embodiments, one can use a probe set that has a first bead and a second bead that comprise identical codeable labels, since the order of the identical codeable labels will be different on the first bead and on the second bead in the aligned detectable complexes (see, e.g., Figure 14(c)).

### Exemplary Embodiments of the Invention

In certain embodiments in which the targets are nucleic acid sequences, the sequence-specific portions of the probes are of sufficient length to permit specific annealing to complementary sequences in target sequences. In certain embodiments, the length of the sequence-specific portion is 12 to 35 nucleotides. Detailed descriptions of probe design that provide for sequence-specific annealing can be found, among other places, in Diffenbach and Dveksler, PCR Primer, A Laboratory Manual, Cold Spring Harbor Press, 1995, and Kwok et al. (Nucl. Acid Res. 18:999-1005, 1990).

In certain embodiments, a probe set according to the present invention comprises a first target-specific probe and a second target-specific probe that adjacently hybridize to the same target sequence. A sequence-specific portion of the first target-specific probe in each probe set is designed to hybridize with the downstream region of the target sequence in a sequence-specific manner (see, e.g., probe A in Fig. 1). A sequence-specific portion of the second target-specific probe in the probe set is designed to hybridize with the upstream region of the target sequence in a sequence-specific manner (see, e.g., probe Z in Fig. 1). The sequence-specific portions of the probes are of sufficient length to permit specific annealing with complementary sequences in target sequences, as appropriate. Under appropriate conditions, adjacently hybridized probes may be ligated together to form a ligation product, provided that they comprise appropriate reactive groups, for example, without limitation, a free 3'-hydroxyl or 5'-phosphate group.

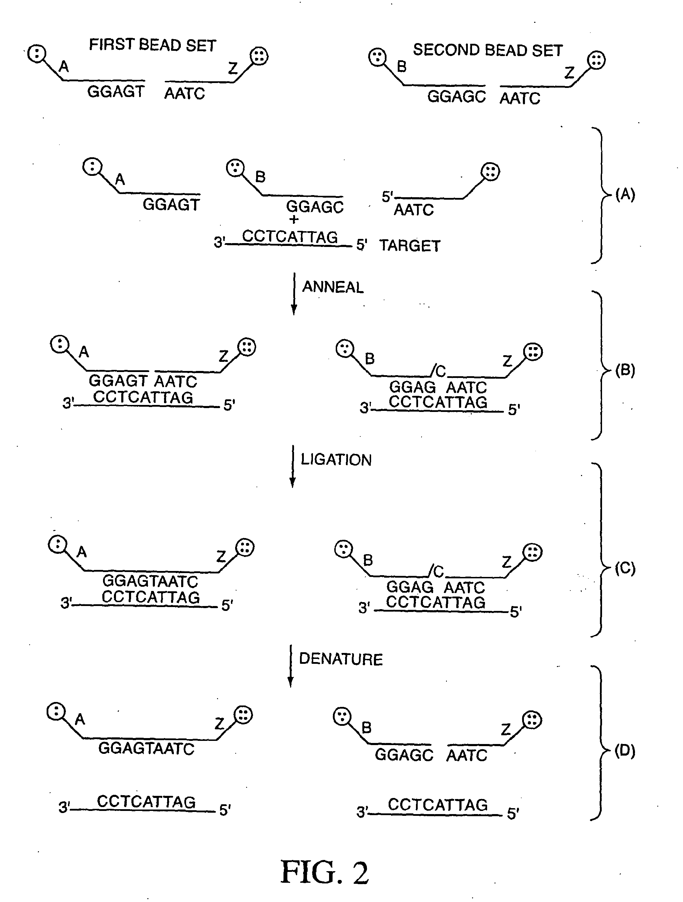

In certain embodiments, two different probe sets may be used to quantitate two different target sequences that differ by one or more nucleotides (see, e.g., Figure 2). According to certain embodiments of the invention, a probe set is designed so that the sequence-specific portion of the first target-specific probe will hybridize with the downstream target region (see, e.g., probe A in Fig. 1, and probes A and B in Fig. 2) and the sequence-specific portion of the second target-specific probe will hybridize with the upstream target region (see, e.g., probe Z in Fig. 1 and Fig. 2). In certain embodiments, a nucleotide base complementary to the pivotal nucleotide, the "pivotal complement," is present on the proximal end of either the first target-specific probe or the second target-specific probe of the probe set (see, e.g., 3' end of probe A in Fig. 1, and the 3' end of probes A and B in Fig. 2).

When the first and second target-specific probes of the probe set are hybridized to the appropriate upstream and downstream target regions, and the pivotal complement is base-paired with the pivotal nucleotide on the target sequence, the hybridized first and second target-specific probes may be ligated together to form a ligation product (see, e.g., Figure 2(b)-(c)). A mismatched base at the pivotal nucleotide, however, interferes with ligation, even if both probes are otherwise fully hybridized to their respective target regions (see, e.g., Figure 2(b)-(c)). Thus, in certain embodiments, highly related sequences that differ by as little as a single nucleotide can be distinguished.

For example, according to certain embodiments, one can distinguish the two potential alleles in a biallelic locus using two different probe sets as follows. The first target-specific probe of each probe set will differ from one another in their pivotal complement, and the codeable labels associated with the two different first target-specific probes will be detectably different (see, e.g., the codeable labels with probes A and B in Fig. 2(a)) Each probe set can also comprise identical second target-specific probes, and the codeable labels associated with the two identical second target-specific probes will be identical (see, e.g., the codeable labels with probe Z in Fig. 2(a)).

One can combine the sample with the two different probe sets. (In certain embodiments, one of the probes of each probe set can further comprise a separating moiety. All three target-specific probes will hybridize with the target sequence under appropriate conditions (see, e.g., Fig. 2(b)). Only the first target-specific probe with the hybridized pivotal complement, however, will be ligated with the hybridized second target-specific probe (see, e.g., Fig. 2(c)). Thus, if only one allele is present in the sample, only one ligation product for that target will be generated (see, e.g., ligation product A-Z in Fig. 2(d)). Both ligation products (A-Z and B-Z) would be formed in a sample from a heterozygous individual.

Further, in certain embodiments, probe sets do not comprise a pivotal complement at the terminus of the first or the second target-specific probe. Rather, the target nucleotide or nucleotides to be detected are located within the sequence-specific portion of either the first target-specific probe or the second target-specific probe. Probes with sequence-specific portions that are fully complementary with their respective target regions will hybridize under high stringency conditions. Probes with one or more mismatched bases in the sequence-specific portion, by contrast, will not hybridize to their respective target region. Both the first target-specific probe and the second target-specific probe must be hybridized to the target for a ligation product to be generated. The nucleotides to be detected may be both pivotal or internal.

In certain embodiments, the first target-specific probes and second target-specific probes in a probe set are designed with similar melting temperatures (Tₘ). In certain embodiments, where a probe includes a pivotal complement, the Tₘ for the probe(s) comprising the pivotal complement(s) of the target pivotal nucleotide sought will be approximately 4-6° C lower than the other probe(s) that do not contain the pivotal complement in the probe set. The probe comprising the pivotal complement(s) will also preferably be designed with a Tₘ near the ligation temperature. Thus, in such embodiments, a probe with a mismatched nucleotide will more readily dissociate from the target at the ligation temperature. In such embodiments, the ligation temperature, therefore, provides another way to discriminate between, for example, multiple potential alleles in the target.

### Certain Exemplary Embodiments of Detecting Targets

The present invention is directed to methods, reagents, and kits for quantitating targets in a sample. In certain embodiments, one detects the presence or absence of target nucleic acid sequences using ligation.

In certain embodiments, for each target nucleic acid sequence to be detected, a probe set, comprising at least one first target-specific probe and at least one second target-specific probe, is combined with the sample and optionally, a ligation agent, to form a ligation reaction mixture. The at least one first probe further comprises a first codeable label comprising at least two labels, and the first codeable label is specific for the first target-specific probe. The at least one second probe further comprises a second codeable label comprising at least two labels, and the second codeable label is specific for the second target-specific probe. The first codeable label is detectably different from the second codeable label.

In certain embodiments, the first and second target-specific probes in each probe set are designed to be complementary to the sequences immediately flanking the pivotal nucleotide of the target sequence (see, e.g., probes A, B, and Z in Fig. 2(a)). Either the first target-specific probe or the second target-specific probe of a probe set, but not both, will comprise the pivotal complement (see, e.g., probe A of Fig. 2(a)). When the target sequence is present in the sample, the first and second target-specific probes will hybridize, under appropriate conditions, to adjacent regions on the target (see, e.g., Fig. 2(b)). When the pivotal complement is base-paired in the presence of an appropriate ligation agent, two adjacently hybridized probes may be ligated together to form a ligation product (see, e.g., Fig 2(c)).

One can then detect the presence or absence of the target nucleic acid sequences by detecting the presence or absence of the ligation product.

In certain embodiments, including, but not limited to, detecting multiple alleles, the ligation reaction mixture may comprise a different probe set for each potential allele in a multiallelic target locus. In certain embodiments, one may use, for example, without limitation, a simple screening assay to detect the presence of three biallelic loci (e.g., L1, L2, and L3) in an individual using six probe sets. See, e.g., Table 1 below.

**Table 1**

| Locus | Allele | Probe Set - Probe (label) | | |
|---|---|---|---|---|
| L1 | 1 | A (2 red), Z (2 blue) | | |
| | 2 | B (4 red), Z (2 blue) | | |
| L2 | 1 | C (2 orange), Y (4 blue) | | |
| | 2 | D (4 orange), Y (4 blue) | | |
| L3 | 1 | E (2 yellow), X (2 green) | | |
| | 2 | F (4 yellow), X (2 green) | | |

In such embodiments, two different probe sets are used to detect the presence or absence of each allele at each locus. The two first target-specific probes of the two different probe sets for each locus, for example, probes A and B for locus L1, comprise the same upstream sequence-specific portion, but differ at the pivotal complement. Also, the two different probes A and B comprise different codeable labels. The two second target-specific probes of the two different probe sets for each locus, for example, probe Z for locus L1, comprise the same downstream sequence-specific portion. Also, the probes Z comprise the same codeable label. (In certain embodiments, one of the probes of each probe set may further comprise a separating moiety, and the other probe of each probe set may not comprise a separating moiety.)

Thus, in embodiments as depicted in Table 1, three probes A, B, and Z, are used to form the two possible L1 ligation products, wherein AZ is the ligation product of the first L1 allele and BZ is the ligation product of the second L1 allele. Likewise, probes C, D, and Y, are used to form the two possible L2 ligation products. Likewise, probes E, F, and X, are used to form the two possible L3 ligation products.

After ligation of adjacently hybridized first and second target-specific probes, one can detect the presence or absence of a ligation product for each of the alleles for each of the loci by detecting the presence of absence of the unique combinations of codeable labels for each allele. For example, one may detect the following combinations of codeable labels: (1) 2 red/2 blue; (2) 4 orange/4 blue; (3) 2 yellow/2 green; and (4) 4 yellow/2 green. Such an individual would be determined to be homozygous for allele 1 at locus L1, homozygous for allele 2 at locus L2, and heterozygous for both alleles 1 and 2 at locus L3.

The person of ordinary skill will appreciate that in certain embodiments, three or more alleles at a multiallelic locus can also be differentiated using these methods. Also, in certain embodiments, more than one loci can be analyzed.

The skilled artisan will understand that in certain embodiments, the probes can be designed with the pivotal complement at any location in either the first target-specific probe or the second target-specific probe. Additionally, in certain embodiments, target-specific probes comprising multiple pivotal complements are within the scope of the invention.

### Detection of Splice Variants

According to certain embodiments, the present invention may be used to identify splice variants in a target nucleic acid sequence. For example, genes, the DNA that encodes for a protein or proteins, may contain a series of coding regions, referred to as exons, interspersed by non-coding regions referred to as introns. In a splicing process, introns are removed and exons are juxtaposed so that the final RNA molecule, typically a messenger RNA (mRNA), comprises a continuous coding sequence. While some genes encode a single protein or polypeptide, other genes can code for a multitude of proteins or polypeptides due to alternate splicing.

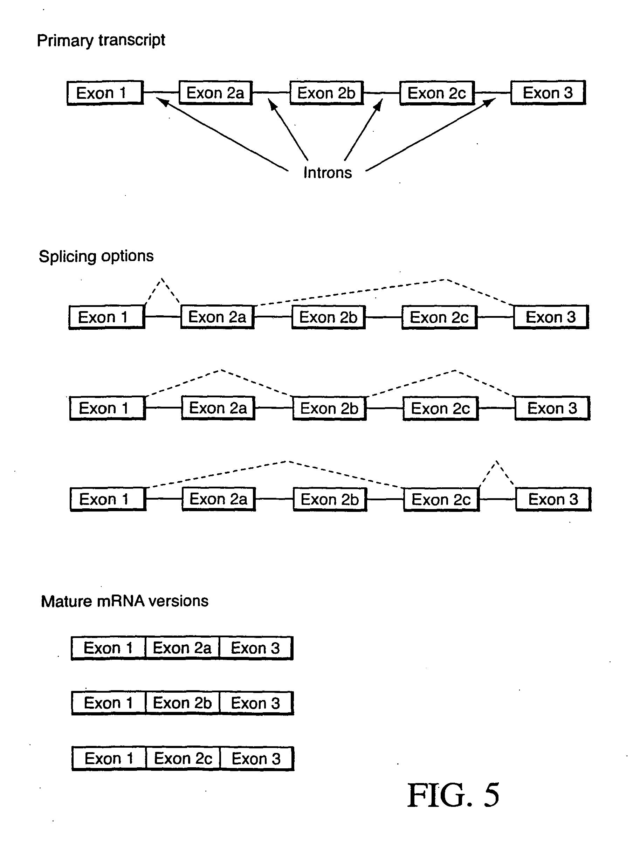

For example, a gene may comprise five exons each separated from the other exons by at least one intron, see Figure 5. The hypothetical gene that encodes the primary transcript, shown at the top of Figure 5, codes for three different proteins, each encoded by one of the three mature mRNAs, shown at the bottom of Figure 5. Due to alternate splicing, exon 1 may be juxtaposed with (a) exon 2a-exon 3, (b) exon 2b-exon 3, or (c) exon 2c-exon 3, the three splicing options depicted in Figure 5, which result in the three different versions of mature mRNA.

The rat muscle protein, troponin T is but one example of alternate splicing. The gene encoding troponin T comprises five exons (W, X, α, β, and Z), each encoding a domain of the final protein. The five exons are separated by introns. Two different proteins, an α-form and a β-form are produced by alternate splicing of the troponin T gene. The α-form is translated from a mRNA that contains exons W, X, α, and Z. The β-form is translated from a mRNA that contains exons W, X, β, and Z.

In certain embodiments, a method is provided for detecting the presence or absence of different splice variants in at least one target nucleic acid sequence in a sample using a different probe set for each different splice variant.

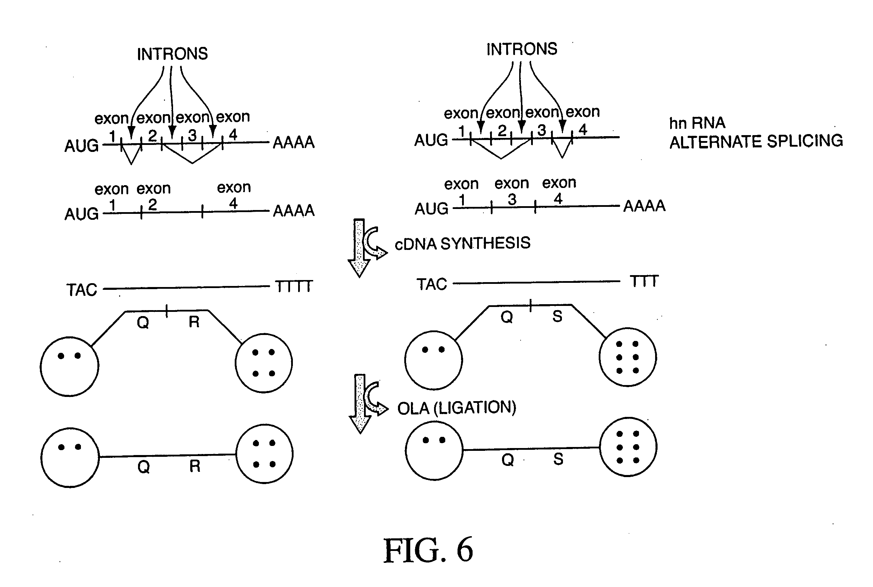

Certain nonlimiting embodiments for identifying splice variants are illustrated by Figure 6. Such embodiments permit one to identify two different splice variants. One splice variant includes exon 1, exon 2, and exon 4 (splice variant E1 E2E4). The other splice variant includes exon 1, exon 3, and exon 4 (splice variant E1 E3E4).

The probe set that is specific for splice variant E1 E2E4 comprises at least one first target-specific probe Q that comprises a sequence-specific portion that hybridizes to at least a portion of exon 1, e.g., it can hybridize to the end of exon 1 that is adjacent to either exon 2 or exon 3. The at least one first target specific probe further comprises a first codeable label. The probe set that is specific for splice variant E1 E2E4 further comprises at least one second target-specific probe R that comprises a sequence-specific portion that hybridizes to at least a portion of exon 2, e.g., it can hybridize to the end of exon 2 that is adjacent to exon 1. The at least one second target-specific probe of the probe set that is specific for splice variant E1 E2E4 further comprises a second codeable label that is detectably different from the first codeable label.

The probe set that is specific for splice variant E1 E3E4 comprises at least one first probe that is the same as the at least one first probe of the probe set that is specific for the splice variant E1 E2E4: The probe set that is specific for splice variant E1 E3E4 further comprises at least one second target-specific probe S that comprises a sequence-specific portion that hybridizes to at least a portion of exon 3, e.g., it can hybridize to the end of exon 3 that is adjacent to exon 1. The at least one second probe of the probe set that is specific for splice variant E1 E3E4 further comprises a second codeable label that is detectably different from the first codeable label and is detectably different from the second codeable label of the at least one second probe of the probe set that is specific for the splice variant E1 E2E4.

After ligation of adjacently hybridized first and second target-specific probes, one can detect the presence or absence of a ligation product for each of the splice variants by detecting the presence or absence of the unique combinations of codeable labels for each splice variant. For example, in certain methods depicted in Figure 6, if the presence of a ligation product with the combination of two dots and four dots is detected, the presence of splice variant E1E2E4 in the sample is detected. If that combination of codeable labels is absent, the absence of splice variant E1E2E4 in the sample is detected. Also, in certain methods depicted in Figure 6, if the presence of a ligation product with the combination of two dots and six dots is detected, the presence of splice variant E1 E3E4 in the sample is detected. If that combination of codeable labels is absent, the absence of splice variant E1E3E4 in the sample is detected.

In certain embodiments, the at least one target nucleic acid sequence comprises at least one complementary DNA (cDNA) generated from an RNA. In certain embodiments, the at least one cDNA is generated from at least one messenger RNA (mRNA). In certain embodiments, the at least one target nucleic acid sequence comprises at least one RNA target sequence present in the sample.

### Methods Employing Addressable Portions

In certain embodiments, one employs unique specifically addressable oligonucleotides, or "addressable portions." Addressable portions are oligonucleotide sequences designed to hybridize to the complement of the addressable portion. For a pair of addressable portions that are complementary to one another, one member will be called an addressable portion and the other will be called a complementary addressable portion.

In certain embodiments, the method comprises forming a ligation mixture comprising a first probe, comprising a first addressable portion and a first target-specific portion; a second probe, comprising a second addressable portion and a second target-specific portion, wherein the first and second target-specific portions are suitable for ligation together when hybridized adjacent to one another on a target; a ligation agent; and a sample. If a target is present in the sample, the first and second target-specific portions of the first and second probes are ligated together to form a ligation product.

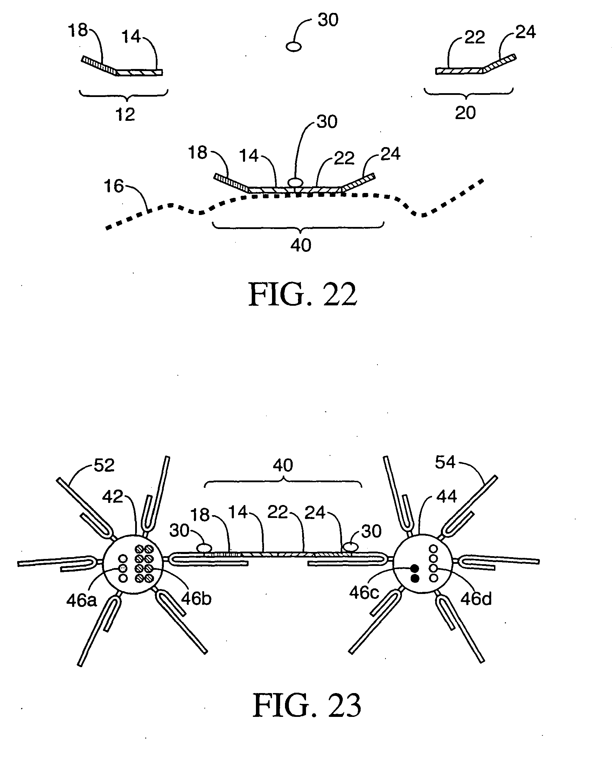

Figure 22 illustrates exemplary embodiments which include a ligation reaction mixture comprising: a first probe 12 that comprises a first target-specific portion 14 and a first addressable portion 18; and a second probe 20 that comprises a second target-specific portion 22 and a second addressable portion 24. The target-specific portions of the probes hybridize to a target 16. The ligation reaction mixture is subjected to a ligation reaction, and the first and second target-specific portions of the first and second probes are ligated together to form a ligation product.

In certain embodiments, after the formation of any ligation products comprising a first addressable portion and second addressable portion, bead pairs are added to the ligation mixture. In certain embodiments, a bead pair comprises a first bead comprising a first complementary addressable portion, wherein the first complementary addressable portion is complementary to the first addressable portion; and a second bead comprising a second complementary addressable portion, wherein the second complementary portion is complementary to the second addressable portion. The first addressable portion of the ligation product hybridizes to the first complementary addressable portion of the first bead, and the second addressable portion of the ligation product hybridizes to the second complementary addressable portion of the second bead, to form a detectable complex comprising the first bead, the ligation product, and the second bead. In certain embodiments, either the first bead or the second bead or both beads may be separation moieties. In certain embodiments, either the first bead or the second bead or both beads may comprise a codeable label.

In certain embodiments, the complementary addressable portion associated with a bead is included in a hairpin structure. In certain embodiments, the hairpin structure comprises a complementary addressable portion and an anchor portion. In certain embodiments, the anchor portion is upstream from the complementary addressable portion, and the anchor portion comprises a first portion and a second portion that complement each other. In certain embodiments, the anchor portion of the hairpin structure is attached to the bead. Certain exemplary embodiments of hairpin structures 52 and 54 are shown in Figure 23.

In certain embodiments, the first and second portions of the anchor portion of the hairpin structure hybridize to one another such that the anchor portion includes one end that is contiguous with the complementary addressable portion and an opposite free end that folds back onto the end contiguous with the complementary addressable portion. See, e.g., Figure 23. In certain embodiments, a first addressable portion and a second addressable portion of a ligation product hybridize to a first complementary addressable portion and a second complementary addressable portion, respectively, that are included in two different hairpin structures that are attached to two different beads to form a detectable complex. See, e.g., Figure 23. In certain embodiments, the hairpin structures are designed such that the free end of the anchor portion is suitable_for ligation together with an adjacent addressable portion of a ligation product that is hybridized to the hairpin structure. See, e.g., Figure 23. In certain such embodiments, the ligation product and the hairpin structure are subjected to a ligation reaction. In such embodiments, the detectable complex comprises the beads and the ligation product that is ligated to the hairpin structures attached to the beads.

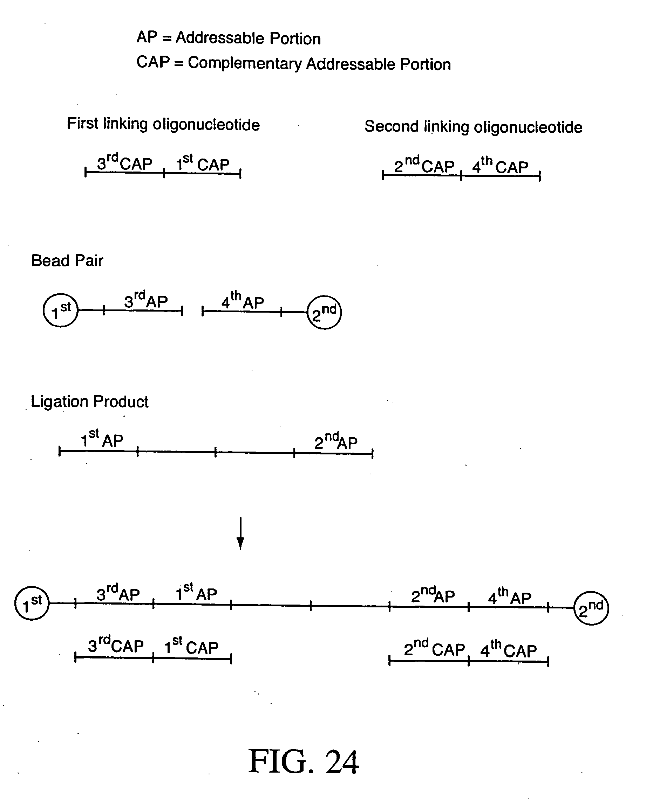

In certain embodiments, after the formation of any ligation products comprising a first addressable portion and second addressable portion, linking oligonucleotide pairs and bead pairs are added to the ligation mixture. Certain such exemplary embodiments are shown in Figure 24. In certain embodiments, a bead pair comprises a first bead comprising a third addressable portion; and a second bead comprising a fourth addressable portion. In such embodiments, the linking oligonucleotide pair comprises a first linking oligonucleotide and a second linking oligonucleotide. The first linking oligonucleotide comprises: a first complementary addressable portion that is complementary to the first addressable portion of the ligation product; and a third complementary addressable portion that is complementary to the third addressable_portion of the first bead. The second linking oligonucleotide comprises: a second complementary addressable portion that is complementary to the second addressable portion of the ligation product; and a fourth complementary addressable portion that is complementary to the fourth addressable portion of the second bead. The first, second, third, and fourth specific addressable portions hybridize to the first, second, third, and fourth specific addressable portions, respectively, to form a detectable complex comprising the ligation product and the beads. In certain embodiments, either the first bead or the second bead or both beads may be separation moieties. In certain_embodiments, either the first bead or the second bead or both beads may comprise a codeable label.

In certain embodiments, the linking oligonucleotides are designed such that after hybridization of the first, second, third, and fourth specific addressable portions to the first, second, third, and fourth specific addressable portions, respectively, adjacent ends of the first and third addressable portions are suitable for ligation together, and adjacent ends of the second and fourth addressable portions are suitable for ligation together. In certain such embodiments, the hybridized ligation product, linking oligonucleotides, and addressable portions of the beads are subjected to a ligation reaction. In such embodiments, the detectable complex comprises the beads and the ligation product that is ligated to the addressable portions of the beads.

In certain embodiments, after the formation of any ligation products comprising a first addressable portion and second addressable portion, the ligation products are separated from excess probes that are not hybridized to a target. In certain such_embodiments, one may employ a filter that is designed such that it captures target and such that unhybridized probes pass through it. Since the ligatation products are hybridized to target, the ligation products will also be captured by the filter. In certain embodiments, one removes the probes that are not hybridized to the target and proceeds with the ligation products.

In certain embodiments, one can separate ligation products from targets by denaturing the ligation product from the target. In certain embodiments, the target can then be destroyed. For example, in certain embodiments in which the target is mRNA, Rnase can be added to decompose the mRNA without damaging the ligation products.

### Single-Bead Assay

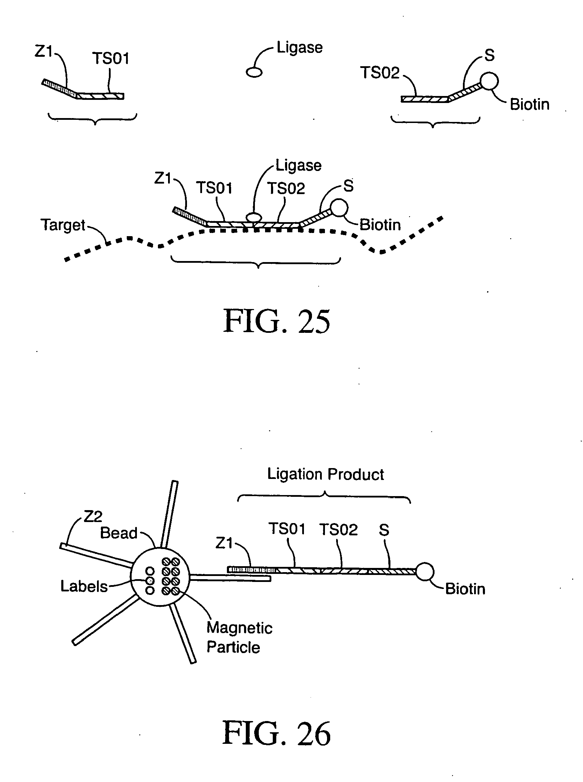

In certain embodiments, the detection of a target may be carried out using two separating moieties and one codeable label. In certain embodiments, the detection uses a probe set comprising a first oligonucleotide probe comprising a first addressable portion (Z1) and a first target-specific portion (TSO1), as shown in Figure 25. In certain embodiments, the probe set further comprises a second oligonucleotide probe comprising a second target specific portion (TS02), a non-specific spacer portion (S), and first separating moiety, such as a biotin molecule, as shown in Figure 25. In certain embodiments, in the presence of the target, the first target-specific portion and the second target-specific portion hybridize to the target such that the two target-specific portions are suitable for ligation. Thus, in certain embodiments, if a target molecule is present, two adjacently hybridized first and second ligation probes may be ligated together to form a ligation product comprising the first and second target-specific portions, the first addressable portion, the spacer portion, and the biotin molecule, as shown in Figure 25.

In certain embodiments, the method employs a bead comprising a codeable label that is attached to one or more oligonucleotides comprising a second addressable portion (Z2). See, e.g., Figure 26. In certain embodiments, the second addressable portion Z2 is complementary to the first addressable portion Z1 such that, if a ligation product is formed, the probe set forms a detectable complex comprising the bead comprising the codeable label, the second addressable portion Z2 hybridized to the first addressable portion Z1, the first and second target-specific portions (TSO1 and TSO2), the spacer portion (S), and the biotin molecule, as shown in Figure 26.

In certain embodiments, the oligonucleotide comprising the second addressable portion Z2 is covalently attached to the bead comprising the codeable label. In certain embodiments, the second addressable portion Z2 may comprise DNA, LNA, PNA, 2'-O-methyl nucleic acid, or any other probe material. In certain embodiments, the sequences of the first and second addressable portions (Z1 and Z2) are optimized to a particular melting temperature or hybridization binding strength.

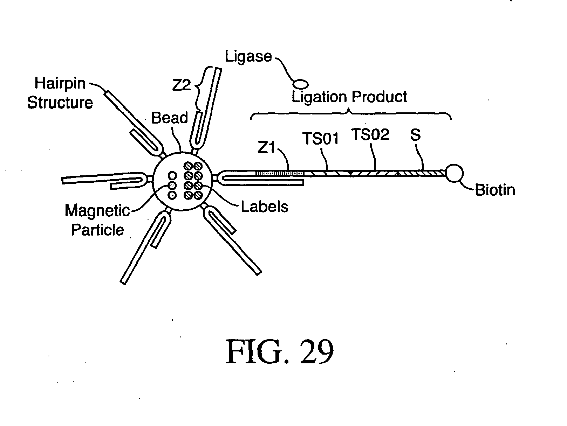

In certain embodiments, the second addressable portion Z2 is part of a hairpin structure attached to the bead comprising the codeable label. See, e.g., Figure 29. In certain embodiments depicted in Figure 29, the hairpin structure is such that, when the first addressable portion Z1 hybridizes to the second addressable portion Z2, the first addressable portion is suitable for ligation to the 3'-end of the hairpin structure. In certain embodiments, the ligation product is ligated to the 3'-end of the hairpin structure to form a detectable complex.

In certain embodiments, the beads comprising the codeable label are polystyrene beads embedded with a magnetic material such as ferrite, making such beads magnetic and denser than certain buffers. In certain embodiments, the codeable labels may be photon-emitting particles embedded or attached to the beads. In certain embodiments, the photon-emitting particles may produce signals at unique wavelengths, and the number of particles for each bead may be varied, allowing different signal intensities and wavelengths to increase the number of unique beads.

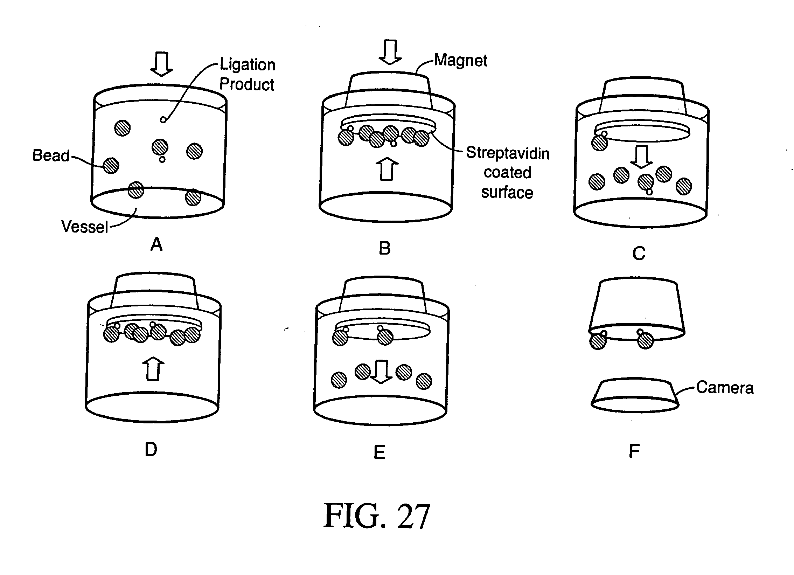

In certain embodiments, after a ligation reaction, the mixture is added to a vessel containing the codeable labels, as shown in Figure 27(A). In certain embodiments, prior to adding the mixture to the vessel containing beads comprising codeable labels, after the ligation reaction, one may substantially remove the first oligonucleotide probe not in a ligation product by chemistry that decomposes probes starting at the 3'-phosphate end. In such embodiments, the ligation product should not be decomposed because the biotin molecule is at the 3'-end. In certain embodiments, targets could be substantially removed by enzymatic digestion, such as with RNase, as a non-limiting example. In certain embodiments, filters may be used to remove RNA or DNA targets.

In certain embodiments shown in Figure 27(B), the detectable complexes are exposed to a streptavidin-coated magnet and the beads are attracted to the magnet. In certain embodiments shown in Figure 27(C), the magnet is turned off, and detectable complexes with biotin molecules remain attached to the streptavidin-coated magnet, while beads comprising codeable labels not in detectable complexes fall away from the streptavidin-coated magnet. In certain embodiments, a second magnet may be placed on the bottom of the vessel, such that the second magnet removes unbound beads from the streptavidin-coated magnet, but does not remove the beads in detectable complexes bound to the streptavidin-coated magnet by a biotin molecule. In certain embodiments shown in Figures 27(D) and 27(E), one repeats the process of turning on the magnet, allowing the detectable complexes to attach to the streptavidin surface, turning off the magnet, and allowing beads comprising codeable labels not in detectable complexes to fall off. In certain embodiments shown in Figure 27(F), the streptavidin-coated magnet is then removed with the bound detectable complexes. In certain embodiments, a camera may be used to evaluate codeable labels to count and identify the detectable complexes attached to the magnet. The number of detectable complexes is then used to quantitate the target molecules in the sample.

In certain embodiments, multiple wells may be used that each contain the same sets of a given number of different beads with codeable labels and different addressable portions. In certain embodiments, each well includes a set of first oligonucleotide probes that have different addressable portions that are complementary to the addressable portions of the different beads. In separate wells, however, the first oligonucleotide probes may have different target specific portions complementary to different targets. In certain such embodiments different multiplex assays may be performed in different wells that employ the same sets of different beads. In certain embodiments, if the number of multiplex assays per well is 1,000, then 1,000 different beads would enable 384,000 different assays in a 384 well plate.

### Quantitation of Targets

In certain embodiments of the invention, one can quantitate the amount of one or more targets, such as target nucleic acid sequences, in a sample. Quantitation can be applied to any of the methods discussed above with respect to detecting the presence or absence of targets. For example, and without limitation, one can quantitate the number of different particular nucleic acid sequences in a sample, including but not limited to, the number of various alleles at one or more loci, the number of particular single nucleotide polymorphisms, and the number of particular splice variants.

In certain embodiments, to quantitate the amount of a target nucleic acid sequence in a sample, one determines the amount of a particular ligation product in a sample by determining the amount of the particular combination of codeable labels for that ligation product. In certain such embodiments, one may determine the quantity of a particular target sequence in a biological sample without subjecting the biological sample to an amplification reaction such as polymerase chain reaction.

Also, in certain embodiments that employ quantum dots as the labels, the number of combinations of sets of quantum dots that are determined correlates directly to the actual number of ligation products in a sample. Thus, in such embodiments, one need not compare the level of intensity of a fluorescent signal to a control signal to evaluate the number of ligation products in the sample.

Quantitation of nucleic acid sequences may have many useful applications. An organism's genetic makeup is determined by the genes contained within the genome of that organism. Genes are composed of long strands or deoxyribonucleic acid (DNA) polymers that encode the information needed to make proteins. Properties, capabilities, and traits of an organism often are related to the types and amounts of proteins that are, or are not, being produced by that organism.

A protein can be produced from a gene as follows. First, the DNA of the gene that encodes a protein, for example, protein "X", is converted into ribonucleic acid (RNA) by a process known as "transcription." During transcription, a single-stranded complementary RNA copy of the gene is made. Next, this RNA copy, referred to as protein X messenger RNA (mRNA), is used by the cell's biochemical machinery to make protein X, a process referred to as "translation." Basically, the cell's protein manufacturing machinery binds to the mRNA, "reads" the RNA code, and "translates" it into the amino acid sequence of protein X. In summary, DNA is transcribed to make mRNA, which is translated to make proteins.

The amount of protein X that is produced by a cell often is largely dependent on the amount of protein X mRNA that is present within the cell. The amount of protein X mRNA within a cell is due, at least in part, to the degree to which gene X is expressed. Whether a particular gene is expressed, and if so, to what level, may have a significant impact on the organism.

For example, the protein insulin, among other things, regulates the level of blood glucose. The amount of insulin that is produced in an individual can determine whether that individual is healthy or not. Insulin deficiency results in diabetes, a potentially fatal disease. Diabetic individuals typically have low levels of insulin mRNA and thus will produce low levels of insulin, while healthy Individuals typically have higher levels of insulin mRNA and produce normal levels of insulin.

Another human disease typically due to abnormally low gene expression is Tay-Sachs disease. Children with Tay-Sachs disease lack, or are deficient in, a protein(s) required for sphingolipid breakdown. These children, therefore, have abnormally high levels of sphingolipids causing nervous system disorders that may result in death.

It is useful to identify and detect additional genetic-based diseases/disorders that are caused by gene over- or under-expression. Additionally, cancer and certain other known diseases or disorders can be detected by, or are related to, the over- or under-expression of certain genes. For example, men with prostate cancer typically produce abnormally high levels of prostate specific antigen (PSA); and proteins from tumor suppressor genes are believed to play critical roles in the development of many types of cancer.

In certain embodiments, using nucleic acid technology, minute amounts of a biological sample can typically provide sufficient material to simultaneously test for many different diseases, disorders, and predispositions. Additionally, there are numerous other situations where it would be desirable to quantify the amount of specific target nucleic acids, e.g., mRNA, in a cell or organism, a process sometimes referred to as "gene expression profiling." When the quantity of a particular target nucleic acid within, for example, a specific cell-type or tissue, or an individual is known, in certain cases one may start to compile a gene expression profile for that cell-type, tissue, or individual. Comparing an individual's gene expression profile with known expression profiles may allow the diagnosis of certain diseases or disorders in certain cases. Predispositions or the susceptibility to developing certain diseases or disorders in the future may also be identified by evaluating gene expression profiles in certain cases. Gene expression profile analysis may also be useful for, among other things, genetic counseling and forensic testing in certain cases. In certain embodiments, gene expression profiles for one or more target nucleic acid sequences may be compiled using the quantitative information obtained according to the inventive methods disclosed herein.

In certain embodiments, when the gene expression levels for several target nucleic acid sequences for a sample are known, a gene expression profile for that sample can be compiled and compared with other samples. For example, but without limitation, samples may be obtained from two aliquots of cells from the same cell population, wherein one aliquot was grown in the presence of a chemical compound or drug and the other aliquot was not. By comparing the gene expression profiles for cells grown in the presence of drug with those grown in the absence of drug, one may be able to determine the drug effect on the expression of particular target genes.

### Protein Detection

In certain embodiments of the invention, methods of detecting the presence or absence of at least two target proteins in a sample are provided. In certain embodiments, the method comprises combining the sample with a different probe set specific for each of the at least two target proteins, each probe set comprising (a) at least one separating bead, comprising a magnetic particle, a first codeable label comprising at least two labels, and a first target-specific probe, wherein the first codeable label is specific for the first target-specific probe, and (b) at least one detecting bead, comprising a second codeable label comprising at least two labels, and a second target-specific probe, wherein the second codeable label is specific for the second target-specific probe. In certain such embodiments, the first and second target-specific probes bind to different portions of the same target protein. In certain embodiments, a detectable complex is formed if the target protein is present in the sample. In certain embodiments, the method further comprises detecting the presence or absence of the at least two different proteins in the sample by counting the detectable complex for each of the at least two target proteins.

In certain embodiments, the target-specific probes are antibodies or fragments of antibodies.

For example, in certain embodiments, the first target-specific probe is a first antibody that can bind specifically to a first portion of a particular target protein, and the second target-specific probe is a second antibody that can bind specifically to a different second portion of the target protein. As a non-limiting example of preparing antibodies for certain such embodiments, one fragment of the target protein is used to generate a first antibody, and a different fragment of the target protein is used to generate a second antibody. The first antibody is attached to a magnetic separating moiety. The second antibody is attached to a codeable label.

In certain embodiments, in the presence of the target protein, the first antibody and second antibody specifically bind to different portions of the target protein, so that binding of either the first antibody or the second antibody to the protein does not inhibit the binding of the other antibody to the protein. If the target protein is present, a detectable complex forms. In certain embodiments, the detectable complex may be separated from unbound antibodies using the separating techniques discussed above. By counting the unique combinations of codeable labels, one detects the presence of absence of particular detectable complexes, which indicates the presence or absence of the target protein in the sample. In certain embodiments, one may determine the quantity of a target protein or proteins in a sample by determining the number of detectable complexes.

### Certain Embodiments of Kits

In certain embodiments, kits for detecting target nucleic acid sequences in a sample are provided. In certain embodiments, the kits comprise a different bead set specific for each of the target nucleic acid sequences. In certain embodiments, each different the bead set comprises (a) at least one separating bead, comprising a magnetic particle, a first codeable label comprising two or more labels, and a first target-specific probe, wherein the first codeable label is specific for the first target-specific probe, and (b) at least one detecting bead, comprising a second codeable label comprising a set of two or more labels, and a second target-specific probe, wherein the second codeable label is specific for the second target-specific probe; and wherein the first codeable label is detectably different from the second codeable label. In certain embodiments, the target-specific probes in each set are suitable for ligation together when hybridized adjacent to one another on a complementary target sequence.

In certain embodiments, the kit comprises a ligation agent. In certain embodiments, the ligation agent is a ligase. In certain embodiments, the ligation agent is a thermostable ligase. In certain embodiments, the thermostable ligase is selected from at least one of T*th* ligase, Taq ligase, and *Pfu* ligase.

### Examples

The following examples illustrate certain embodiments of the invention, and do not limit the scope of the invention in any way.

### Example 1

The following experiment showed that probes that were attached to beads hybridized to a target nucleic acid sequence and were ligated in an oligonucleotide ligation assay (OLA). The amount of ligated product that was produced with probes attached to beads was compared to the amount of ligation product produced by the same probes that were not attached to beads.

Magnetic beads were attached to oligonucleotide probes. Magnetic beads coated with streptavidin were obtained from Seradyne (Sera-Mag., Lot No. 113564). Biotin was attached to oligonucleotide probes (target-specific oligonucleotides (TSO probes)) as follows. Biotin-labeled oligonucleotide probes (target-specific oligonucleotide probes (TSO probes)) were synthesized by Applied Biosystems, Inc. (Foster City, CA). The TSO probes were designed to hybridize to a target nucleic acid sequence. The sequence of the TSO probe is given in Table 2 below. Approximately 100 µl of the streptavidin-coated magnetic beads (1 mg/ml) were added to 100 µl of a biotin-TSO probes (10 µM) into 0.2 ml tubes. The mixture was incubated for 60 minutes at 4°C, allowing the biotin to bind to the streptavidin.

**Table 2**

| | |
|---|---|
| TSO (SEQ ID 1) | TAC GGA TGC TCA CTA CGC TAG GTT TTT TTT TTT TTT TTT T |
| pTSO (SEQ ID 2) | TTT TTT TTT TTT TTT TTT TTA TGC CTC GTG ACT GCT ACC A |
| synthetic target (SEQ ID 3) | AAA AAA AAA AAA AAA AAA CCT AGC GTA GTG AGC ATC CGT ATG GTA GCA GTC ACG AGG CAT AAA AAA AAA AAA AAA AAA AA |
| 399 -Taqman (SEQ ID 4) | TTT TTT TTT TTT TTT TTT TTA TGC CTC GTG ACT GCT ACC ATA CGG ATG CTC ACT ACG CTA GGT TTT TTT TTT TTT TTT TT |

The magnetic beads were then washed to remove unbound TSO probes from the beads as follows. Forty µl of PBS buffer was added to the 20 µl of the mixture in each of the 0.2 ml tubes. The PBS buffer comprised:

| | |
|---|---|
| KPO4 (dibasic) | 1.82 g/l |
| NaPO4 (monobasic) | 0.22 g/l |
| NaCl | 8.76 g/l |
| adjusted to pH 7.4. | |

The sample was vortexed briefly then placed on top of a magnet for 4 minutes. After 4 minutes, 40 µl was removed from each of the 0.2 ml tubes while the 0.2 ml tubes were still on the magnet. The 40 µl of supernatant from each tube was stored in a 1 ml tube for later analysis. The magnetic separation and washing were repeated 3 more times, for a total of 4 magnetic separations and washings.

A second oligonucleotide probe (pTSO probe) was designed to hybridize to the target nucleic acid sequence next to the region that is complementary to the TSO probe sequence. The pTSO probe is adjacent to the TSO probe when both TSO and pTSO probes hybridize to the target nucleic acid sequence. When both TSO and pTSO probes hybridize to the target nucleic acid sequence they can be ligated together in a ligation reaction. Biotin-labeled pTSO probes were synthesized by Applied Biosystems, Inc. (Foster City, CA). The sequence of the pTSO probe is given in Table 2.

Fluorescent beads coated with streptavidin were obtained from Bangs Laboratories (Fisher, IN).

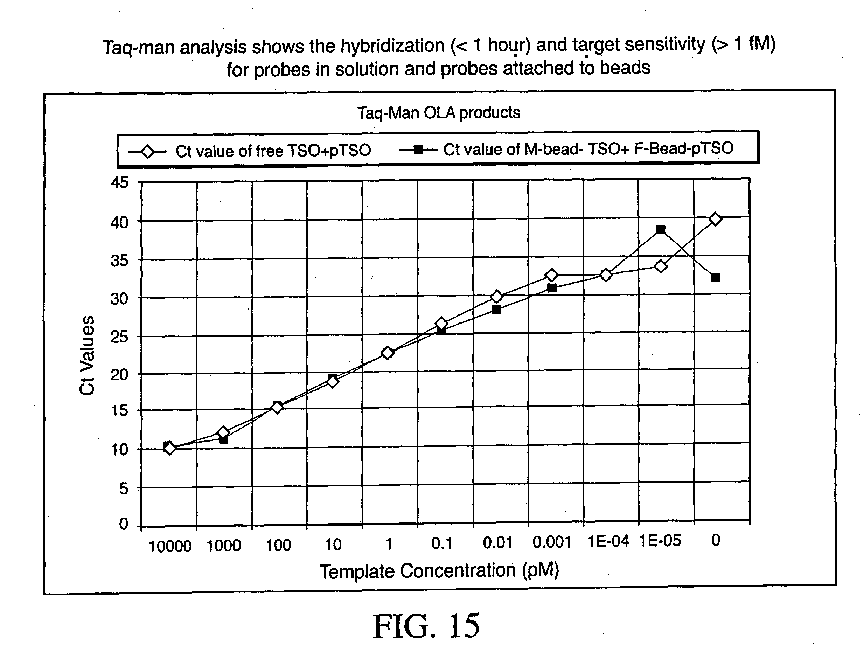

Ligation reactions (OLA) were performed. Eleven different reactions were prepared by mixing the magnetic beads with the attached TSO probes, the fluorescently-labeled streptavidin-coated beads, the pTSO probes with biotin attached, a ligase, and the synthetic target nucleic acid in a different concentration for each reaction. The sequence of the synthetic target is given in Table 2. The synthetic target nucleic acid sequence concentrations in each of the 11 reactions, descending in orders of magnitude, ranged from 10 nM to as little as 10 aM (see Figure 15 for actual concentrations). There was one control with no synthetic target nucleic acid sequence.

The recipe for the reactions was as follows:
2 µl 10x Taq ligase buffer
2 µl biotin-labeled pTSO probe (100 nM)
2 µl Fluorescent beads (100 µg/ml)
16 µl magnetic beads attached to TSO (1 mg/ml)
0.5 µl ligase (40 U/µl)
2 µl synthetic target (10 nM - 10 aM)
The ligation reactions were incubated at for ten cycles of 15 seconds at 95 °C, then cooled to 50 °C for 20 minutes on an ABI 9700 Thermal Cycler. Taq Ligase and ligase buffer were obtained from New England Biolabs (Catalog No. M0208).

After the ligation reaction, the magnetic beads were washed to remove any unligated fluorescent beads. Forty µl of PBS buffer was added to the 20 µl of the mixture in each of the 0.2 ml tubes. The sample was vortexed briefly then placed on .. top of a magnet for 4 minutes. After 4 minutes, 40 µl was removed from each of the 0.2 ml tubes while the 0.2 ml tubes were still on the magnet. The 40 µl of supernatant from each tube was stored in a 1 ml tube for later analysis. The magnetic separation and washing were repeated 3 more times, for a total of 4 magnetic separations and washings.

In addition to the 11 OLA reactions including beads, 11 other OLA reactions, were performed without beads using the same TSO and pTSO probes without beads, the same ligase, the same ligase buffer, and the same concentrations of the target nucleic acid sequence. The recipe for those reactions was as follows:
2 µl 10x ligase buffer
11.5 µl water
2 µl biotin-labeled TSO probe (100 nM)
2 µl biotin-labeled pTSO probe (100 nM)
0.5 µl ligase (40 U/µl)
2 µl synthetic target (10 nM - 10 aM)

Magnetic beads from each of the reactions of varying concentration were then removed from the reactions with a magnet. The beads were washed as described above and transferred to a separate detection vessel for each different reaction. The detection vessel was a Petroff-Hausser counting chamber (VWR, catalog No. 15170-048). The fluorescent beads of the ligation products were then detected with an ABI 7700 (Applied Biosystems, Foster City, CA).

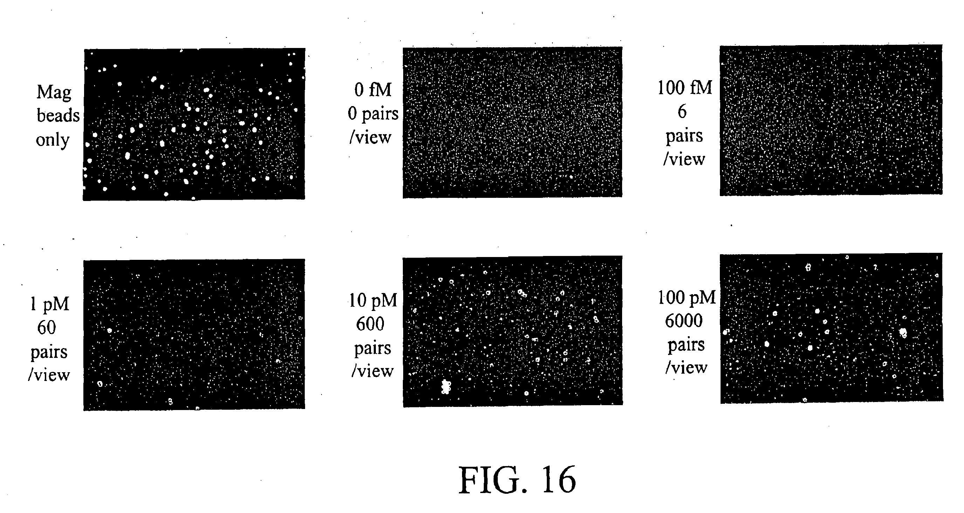

Figure 16 shows five photographs of bead pairs (containing fluorescent beads) visible from five of the reactions and a photograph of magnetic beads only by visible light microscopy. One "view" in each panel represents approximately 0.5 µl average volume of the ligation products. Figure 16 shows that the fluorescent beads have been successfully paired to magnetic beads, washed, and transferred.

OLA reactions with the beads attached to probes were compared to each of the counterpart target concentration OLA reactions without beads attached to probes. The ligation products were measured with a Taqman^{™} analysis.

The 399-Taqman probe was provided by Applied Biosystems (Foster City, California). The sequence of the probe is given in Table 2. The Taqman^{™} probes and procedures for using them are described in, e.g., U.S. Pat. No. 5,538,848. Taqman^{™} probes work by the 5'-nuclease activity of a DNA polymerase. A Taqman^{™} probe hybridizes to a target nucleic acid sequence if the target is present. The Taqman probe has a fluorescent molecule on one end of the probe, and a quenching molecule at the other end of the probe. When the probe is intact with both the fluorescent molecule and quenching molecule attached, there is no fluorescence.

Primers are added that also hybridize to the target nucleic acid sequence. A polymerization reaction is then started at the primer, which adds nucleotides to the end of the primer. The Taqman^{™} probe on the target nucleic acid sequence is cleaved during the polymerization reaction as a result of the strand replacement that occurs during DNA polymerization. That cleavage frees the fluorescent molecule from the presence of the quenching molecule on the probe, which results in the fluorescent molecule fluorescing. Thus, the detection of fluorescence indicates the presence of the particular target nucleic acid sequence involved in the polymerase reaction.

Moreover, the level of fluorescence correlates to the amount of target nucleic acid sequence in a sample (the higher the level of fluorescence, the higher the amount of target nucleic acid sequence). A Ct value for the level of fluorescence for a given sample can be calculated. Ct values are inversely related to the level of fluorescence. In other words, the lower the Ct value, the higher the level of fluorescence.

The recipe for the Taqman^{™} analysis on each of the products from the 22 different OLA reactions was as follows
1 µl 399-Taqman™ probe (5 µM)
2.5 µl 116/115 primers (10 µM)
6.5 µl water
12.5 µl 2x Master Mix
2.5 µl sample from the previous OLA reaction
The Taqman^{™} reactions were incubated at 95°C for ten minutes, followed by cycles comprising a first step of 15 seconds at 95°C, and a second step of 1 minute at 60°C. After a certain number of cycles, a signal appears. The number of cycles a reaction undergoes before a signal appears is recorded and referred to as the Ct value. The sequences of the 116/115 primers for the Taqman^{™} reactions are given in Table 2. Master Mix was obtained from Applied Biosystems (Cat. No. 4318739).

Taqman assays of the products of each of the OLA reactions with the beads present were compared to the products of the counterpart target nucleic acid sequence concentration OLA reactions without the beads. The results are shown in Figure 15. The Taqman analysis in Figure 15 shows the hybridization (less than one hour) and target sensitivity (greater than 1 fM) for probes in solution and probes attached to beads. The detection occurred with a reaction time of about 1 hour.

### Example 2

The following experiment was performed to determine the number of bead pairs that were detected when different amounts of synthetic target molecules were used.

Polystyrene beads were obtained from Bangs (No. PA05N/2057). The beads were approximately 3.1 µm in diameter, had -NH₂ groups on the surface at a density of 10⁵ sites per µm² (according to the manufacturer), and had a density of 1.073 g/cm³.

As discussed in Example 1, the TSO probe was designed to hybridize to a target nucleic acid sequence. The sequence of the TSO probe is given in Table 2. The TSO probe was attached at the 5' end to the amine groups on the Bangs beads, to create TSO-beads, according to the following protocol.

The beads (1.0 ml at 100 mg/ml) were washed twice in 10.0 ml of PBS as previously described. After the second wash, the beads were resuspended in 10.0 ml of glutaraldehyde solution (10% glutaraldehyde in PBS). The beads were allowed to react at room temperature for two hours with continuous mixing. The beads were then washed twice in PBS as previously described, and resuspended in 5 ml of PBS. The amine-coupled TSO probe was placed in 5 ml of PBS and combined with the 5 ml solution containing the beads. The mixture was allowed to react at room temperature for 2-4 hours with continuous mixing. The beads were then washed again, and resuspended in 10 ml of PBS containing 0.1 % Tween-20. The resuspended beads were incubated for 30 minutes. The beads were then washed once more, and resuspended in a storage 10 ml of PBS containing 0.1 % Tween-20.

As discussed above, a second oligonucleotide probe (pTSO probe) was designed to hybridize to the target nucleic acid sequence next to the region that is complementary to the TSO probe sequence. The pTSO probe is adjacent to the TSO probe when both the TSO and pTSO probes hybridize to the target nucleic acid sequence. When both TSO and pTSO probes hybridize to the target nucleic acid sequence they can be ligated together in a ligation reaction. Biotin-labeled pTSO probes were synthesized by, and obtained from Applied Biosystems, Inc. (Foster City, CA). The sequence of pTSO is given in Table 2.

Oligonucleotide Ligation Assay (OLA) reactions were performed. Eight different reactions were prepared by mixing the TSO-beads with the biotin-labeled pTSO, ligase, and synthetic target at varying concentrations. The reaction mixture was as follows:

| | |
|---|---|
| 2 µl | 10x ligase buffer |
| 2 µl | biotin-labeled pTSO (100nM) |
| 2 µl | TSO-beads |
| 14 µl | ddH₂O |
| 0.25 µl | ligase (40 U/µl) |
| 2 µl | synthetic target (at varying concentration of 1 nM down to 1 fM, plus one reaction with no target) |

The ligation reactions were incubated at for ten cycles of 15 seconds at 95 °C, then cooled to 50 °C for 20 minutes on an ABI 9700 Thermal Cycler. Taq Ligase and ligase buffer were obtained from New England Biolabs (Catalog No. M0208).

Streptavidin-coated magnetic beads were obtained from Seradyne (Sera-Mag., Lot No. 113564) which were made of polystyrene containing 40% magnetite (Fe₃O₄), were approximately 1.0 µm in diameter, and had a density of 1.5 g/cm². The streptavidin was on the surface of the beads at a density of 10⁷ sites per bead.

In separate reactions, 20 µl of each of the eight different OLA reactions were added to 20 µl of the streptavidin-coated beads (10⁶ beads/µl). These mixtures were incubated at 4°C for 1 hour.

After incubation, each of the 40 µl OLA streptavidin-coated bead mixtures were vortexed briefly, then sonicated for 10 seconds at power level 9 on a VWR "Aquasonic" ultrasonic cleaner. After sonication, 20 µl of each of the mixtures were placed into separate 0.2 ml tubes then subjected to magnetic separation and washing.

The procedure for magnetic separation and washing was as follows. Forty µl of PBS buffer was added to the 20 µl of the mixture in each of the 0.2 ml tubes. The sample was vortexed briefly then placed on top of a magnet for 4 minutes. After 4 minutes, 40 µl was removed from each of the 0.2 ml tubes while the 0.2 ml tubes were still on the magnet. The 40 µl of supernatant from each tube was stored in a 1 ml tube for later analysis. The magnetic separation and washing were repeated 3 more times, for a total of 4 magnetic separations and washings.

After the magnetic separation and washing, each of the supernatants collected in the 1 ml tubes were centrifuged for 5 minutes.

A portion of the remainder of each of the eight different OLA reactions left in the 0.2 ml tubes after magnetic separation and washing was subjected to Taqman^{™} analysis to determine the number of ligated bead pairs in each reaction. In addition, Taqman^{™} analysis was performed on a portion of each of the supernatants in the 1 ml tubes to determine how many bead pairs were not separated from the wash buffer by the magnetic separation procedure.

The 399-Taqman probes were provided by Applied Biosystems (Foster City, California). The sequence of the probe is given in Table 2. In general, Taqman^{™} probes and procedures for using them are described in, e.g., U.S. Pat. No. 5,538,848. Taqman^{™} probes work by the 5'-nuclease activity of a DNA polymerase. A Taqman^{™} probe hybridizes to a target nucleic acid sequence if the target is present. The Taqman probe has a fluorescent molecule on one end of the probe, and a quenching molecule at the other end of the probe. When the probe is intact with both the fluorescent molecule and quenching molecule attached, there is no fluorescence.

Primers are added that also hybridize to the target nucleic acid sequence. A polymerization reaction is then started at the primer, which adds nucleotides to the end of the primer. The Taqman^{™} probe on the target nucleic acid sequence is cleaved during the polymerization reaction as a result of the strand replacement that occurs during DNA polymerization. That cleavage frees the fluorescent molecule from the presence of the quenching molecule on the probe, which results in the fluorescent molecule fluorescing. Thus, the detection of fluorescence indicates the presence of the particular target nucleic acid sequence involved in the polymerase reaction.

Moreover, the level of fluorescence correlates to the amount of target nucleic acid sequence in a sample (the higher the level of fluorescence, the higher the amount of target nucleic acid sequence). A Ct value for the level of fluorescence for a given sample can be calculated. Ct values are inversely related to the level of fluorescence. In other words, the lower the Ct value, the higher the level of fluorescence.

The recipe for the Taqman^{™} analysis on each of the products from the 8 different OLA reactions and 8 saved supernatants was as follows:
1 µl 399-Taqman™ probe (5 µM)
2.5 µl 116/115 primers (10 µM)
6.5 µl water
12.5 µl 2x Master Mix
2.5 µl sample from the previous OLA reaction
The Taqman^{™} reactions were incubated at 95°C for ten minutes, followed by cycles comprising a first step of 15 seconds at 95°C, and a second step of 1 minute at 60°C. The sequences of the 116/115 primers for the Taqman^{™} reactions are given in Table 2. Master Mix was obtained from Applied Biosystems (Cat. No. 4318739).

In addition, portions of the bead pairs from each reaction and from each of the supernatants collected were plated separately on grids. The grids were visually inspected in order to calculate the total number of bead pairs in each of the supernatants and in each of the reactions.

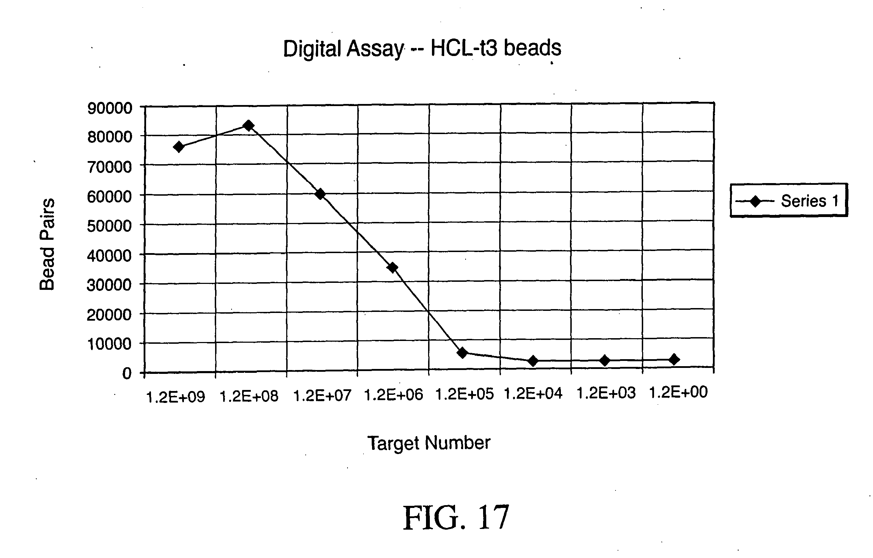

The results of the Taqman^{™} analyses and visual inspections are shown in Figure 17 and in Tables 3, 4, and 5.

### Number of Bead-Pairs and Beads in Each Reaction As Determined by Visual Inspection

**TABLE 3**

| Target Conc. | Target No. | Bead pairs per grid | Total No. Bead pairs | % pairs per target | Unpaired beads per grid |
|---|---|---|---|---|---|
| 100 pM | 1.2x10⁹ | 191 | 76400 | 0.0063 | 1 |
| 10 pM | 1.2x10⁸ | 208 | 83200 | 0.07 | 0 |
| 1 pM | 1.2x10⁷ | 150 | 60000 | 0.50 | 20 |
| 100 fM | 1.2x10⁶ | 85 | 34000 | 2.8 | 40 |
| 10 fM | 1.2x10⁵ | 11 | 4400 | 3.7 | 7 |
| 1 fM | 1.2x10⁴ | 4 | 1600 | 13 | 5 |
| 100 aM | 1.2x10³ | 4 | 1600 | 133 | 3 |
| 0 | 0 | 3 | 1200 | NA | 9 |

Table 3 shows the total number of bead pairs counted in the grid for each reaction, and the number of bead pairs calculated to have been magnetically separated in each reaction in view of the percentage of the reaction material placed on the grid. It also shows the calculated percentage of bead pairs generated per target molecule present in the sample, and the number of beads not incorporated into a bead pair.

### Calculated Number of Successful Ligations Determined by Taqman Analysis

**TABLE 4**

| Target Conc. | Pre-separation Ct | Pre-separation yield (fM) | No. ligations before separation | % ligations per target before separation | Post-separation Ct | Post-separation yield fM) |
|---|---|---|---|---|---|---|
| 100 pM | 16.35 | 10157 | 1.2 x 10⁸ | 10 | 16.66 | 7917.0 |
| 10pM | 15.28 | 23579 | 2.8 x 10⁸ | 236 | 15.9 | 14412.9 |
| 1 pM | 17.17 | 5328.8 | 6.4 x 10⁷ | 533 | 17.28 | 4870.5 |
| 100 fM | 20.43 | 407.2 | 4.9 x 10⁶ 407 | | 20.98 | 264.8 |
| 10 fM | 22.65 | 70.6 | 8.5 x 10⁵ | 709 | 25.92 | 5.4 |
| 1 fM | 23.71 | 30.8 | 3.7 x 10⁵ | 3078 | 27.86 | 1.2 |
| 100 aM | 23.99 | 24.6 | 3.0 x 10⁵ | 24560 | 29.43 | 0.3 |
| 0 | NA | NA | | | 28.63 | 0.6 |

Table 4 shows the calculated yield of ligation products as determined by Taqman analysis. Table 4 shows the concentration and number of ligation products calculated to have been present before and after the magnetic separation procedures, and the calculated percentage of ligations generated per target in the sample.

### Number of Ligations After Separation by Taqman Analysis

**TABLE 5**

| Target Conc. | No. ligations after separation | % ligations after separation |
|---|---|---|
| 100 pM | 9.5 x 10⁷ | 78 |
| 10pM | 1.7 x 10⁸ | 61 |
| 1 pM | 5.9 x 10⁷ | 91 |
| 100 fM | 3.2 x 10⁶ | 65 |
| 10 fM | 6.5 x 10⁴ | 8 |
| 1 fM | 1.4 x 10⁴ | 4 |
| 100 aM | 4.1 x 10³ | 1 |
| 0 | 7.7 x 10³ | NA |

Table 5 shows the number of ligation products calculated to have been present in each of the reactions after the separation procedures. Table 5 also shows the calculated percentage of ligations generated in each of the OLA reactions that were separated from the wash buffer and other reactants by the magnetic separation procedures. The number of ligation products were determined by Taqman assays.

### Example 3

Coded polystyrene beads of approximately 5.6 µm diameter were purchased from Luminex (Luminex Corp., Austin, TX). The beads purchased had been impregnated with two colors of dye, and possess different intensities of dye, creating unique optical code detectable by a Luminex 100 Flow Cytometer. Oligonucleotides were attached to the surface of the beads using the NH₂ ester chemistry described in the Luminex manuals. One poly ethylene glycol (PEG) linker was included between the amine group on the Luminex bead and 5'-end of the attached oligonucleotide. The oligonucleotide comprised a 20 base primer sequence and a 20 base long sequence (referred to as an addressable portion) that was designed for specific hybridization with minimal cross reactivity at a specific temperature. The primer sequence was located between the PEG linker and the addressable portion. After the oligonucleotides were attached, the beads were washed four times with a phosphate buffered saline PBS, as described in Example 1 above, with 0.1 % Tween-20 at pH 7.4, in order to remove oligonucleotides not covalently attached to beads. The washing was between 50 - 65°C, and lasted 20 minutes. Sequences of the two different oligonucleotides with two different addressable portions that were attached to the two differently coded beads are designated Z1 and Z2 as shown in Table 6 below. The addressable portions of the sequences of Z1 and Z2 are shown in bold.

**TABLE 6**

| | |
|---|---|
| Z1 (SEQ ID 5) | NH₂-PEG-GCTGATGCTACTGGATCGCT **ACCGTGACCCTTCCGA** |
| Z2 (SEQ ID 6) | NH2-PEG-GCTTGCCTGCTCGACTTAGA AATCGGTCTCGTCCTTCA |
| ASO1 (SEQ ID 7) | p-**GCGTAATCGTTGCTTCATAG** CCTGGCAGTAAATTCTA G |
| ASO2 (SEQ ID 8) | p-**ACAGCAGTGAGTCTTTAGG** CCTGGCAGTAAATTCTA C |
| Z3 (SEQ ID 9) | CTATGAAGCAACGATTACGC TCGGAAGGGTCACGGT |
| Z4 (SEQ ID 10) | CCTAAAGACTCACTGCTGT TGAAGGACGAGACCGATT |
| LSO (SEQ ID 11) | p-CTCAACCTTACTTGAGGC TGGTAGCAGTCACGAGGCAT- PEG-BIO |

A set of first Luminex beads (B1) and a set of second Luminex beads (B2) (10,000 beads per set) were used. The first bead (B1) comprised a first codeable label and oligonucleotide Z1. The second bead (B2) comprised a second codeable label and oligonucleotide Z2.

Ligation probes were used as follows. Two probes were allele specific oligos (ASO1 and ASO2, shown in Table 6) which comprised a target specific portion complementary to the target sequence containing a particular single nucleotide polymorphorism (SNP). The ASO's were designed with the base complementary to the SNP located at the 3' end of the probe. Thus, ASO1 and ASO2 had different bases at their 3'-ends.

ASO1 comprised an addressable portion 5' to the target specific portion. ASO2 comprised a different addressable portion 5' to the target specific portion. Both ASO's were 40 bases in length. The twenty bases at the 3'-ends of the ASO's were target specific portions, while the 20 bases at the 5'-ends were addressable portions. The addressable portions of ASO1 and ASO2 are shown in bold in Table 6.

A locus specific oligo (LSO) was also provided. The sequence of LSO is shown in Table 6. LSO comprised a target specific portion complementary to the target adjacent to the part of the target that was complementary to the target specific portions of the ASOs, such that LSO and ASO1 or ASO2, when hybridized to the target, were suitable for ligation together. LSO had a biotin molecule attached to the 3' end with a PEG linker. The PEG linker was disposed between the last 3' nucleotide and the biotin molecule. LSO was 40 bases in length. Twenty bases at the 5'-end of the LSO were the target specific portion, while the 20 bases at the 3'-end of LSO were complementary to a particular Taqman primer.

A synthetic template Z3 comprised a 3'-end sequence complementary to the addressable portion of oligonucleotide Z1 on bead B1, and comprised a 5'-end sequence complementary to the addressable portion on ASO1, such that ASO1 and oligonucleotide Z1 are suitable for ligation when hybridized to synthetic template Z3.

A synthetic template Z4 comprised a 3'-end sequence complementary to the addressable portion of oligonucleotide Z2 on bead B2, and comprised a 5'-end sequence complementary to the addressable portion on ASO2, such that ASO2 and oligonucleotide Z2 are suitable for ligation when hybridized to synthetic template Z4.

The target was genomic DNA heated for 30 minutes at 100°C. The target was known (verified by other means) to contain a single nucleotide polymorphism (SNP) complementary to ASO1 but not complementary to ASO2.

The ligation reaction volume was 6.75 microliters. The reaction mixture was as follows.

| | |
|---|---|
| 0.5 µl | 10x Ligase Buffer |
| 0.5 µl | ASO1 probes at 100 nM (3x10¹⁰ probes) |
| 0.5 µl | ASO2 probes at 100 nM (3x10¹⁰ probes) |
| 0.5 µl | LSO probes at 100 nM (3x10¹⁰ probes) |
| 0.5 µl | Template Z3 at 10 nM concentration (3x10⁹ molecules) |
| 0.5 µl | Template Z4 at 10 nM concentration (3x10⁹ molecules) |
| 1 µl | solution of B1 (10,000 beads) |
| 1 µl | solution of B2 (10,000 beads) |
| 0.25 µl | Taq Ligase (40 units/µl) |
| 0.5 µl | heated gDNA (100 ng/µl) |

Taq Ligase and 10X ligase buffer were purchased from New England Biolabs (Catalog No. M0208). Beads were sonicated for 10 seconds before being added to the reaction mixture. The reaction mixture was temperature cycled, starting at 50 °C for 5 minutes to hybridize and ligate the ASO and LSO probes on the target, and to ligate the Z1 or Z2 oligonucleotides to the ASO probes on the synthetic templates. Then, the temperature was increased to 85 °C for 15 seconds, in order to denature the probes from the target, and to denature the synthetic templates from the probes and oligonucleotides. This cycle was repeated 100 times. At the completion of the temperature cycles, the beads were washed 3 times in PBS buffer 0.1 % Tween-20 at 85 °C.

Approximately 10 million, 1 µm diameter streptavidin-coated magnetic beads were mixed with the reaction mixture after the ligation reaction. The beads were purchased from Seradyn Inc., and were made of polystyrene with 40% magnetite (Fe₃O₄), had a density of 1.5 g/cm³, and had a streptavidin coating (10⁵ - 10⁶ streptavidin molecules per bead). Prior to use, the magnetic beads were washed 5 times with PBS buffer 0.1% Tween-20 and 0.1 % BSA and sonicated for 10 seconds. The binding buffer used to bind the magnetic beads to biotin molecules was PBS with 0.1 % Tween-20 in a volume of 20 µl (10µl reaction mixture from the ligation reaction, 10ul magnetic beads). The 20 µl volume of magnetic beads and reaction mixture from the ligation reaction formed the binding mixture, and was incubated in a tube for two hours at room temperature (25°C) with continuous rotation of the tube. If the streptavidin on a magnetic bead bound to the biotin on an LSO, a bead pair was formed.

After the binding mixture was incubated, the binding mixture was sonicated for 10 seconds. The binding mixture was pipetted into a first tube that was sitting in a second tube filled with a high-density solution (∼1.3 g/cm³) of 6X SSC (90 mM Na Citrate, 0.9 M NaCl, pH 7.0) and 0.1% Bovine Serum Albumin (New England Biolabs). The first tube had no bottom such that it was partially filled with the high-density solution. The lower-density (∼1.05 g/cm³) binding buffer of the binding mixture remained on top of the higher density fluid without significant mixing. A magnet located below the tubes attracted the magnetic beads to the bottom of the second tube.

Luminex beads that were not paired to magnetic beads remained toward the top of the first tube and were disposed of by removing the first tube from the second tube. The removal was performed by covering the top of the first tube, then lifting the first tube out of the second tube. Luminex beads in detectable complexes that had been pulled down by the magnetic beads were then released from the magnet, and vortexed into a homogenous solution. The homogeneous solution was then aspirated into a flow cytometer. The Luminex beads were read one at a time, and their identity determined by the unique codes on the beads (B1 or B2). Almost all the Luminex beads were B1 beads, indicating the presence of the SNP corresponding to ASO1 (data not shown).

Disclosed is also:
A method for quantitating a target comprising; forming a reaction mixture comprising: a sample possibly containing the target; a codeable label; one or more target-specific probes, wherein each target-specific probe binds specifically to the target under selective binding conditions; and a separating moiety; treating the reaction mixture under reaction conditions such that a detectable complex is produced when the target is present, and wherein the detectable complex comprises the codeable label, the target-specific probe, and the separating moiety; and quantitating the target by counting the number of codeable labels.
Disclosed is also the above method, further comprising separating the detectable complex from codeable labels that are not included in the detectable complex after treating the reaction mixture and before quantitating the target.
Disclosed is also a method for quantitating at least two different particular targets comprising; forming a reaction mixture comprising: a sample possibly containing two or more different particular targets; a different codeable label specific for each different particular target; one or more different target-specific probes specific for each different particular target that bind specifically to the target under selective binding conditions; and a separating moiety; treating the reaction mixture under reaction conditions such that when a particular target is present, a detectable complex is produced, which comprises the codeable label specific for the particular target, the target-specific probe specific for the particular target, and the separating moiety; and quantitating each of the different particular targets by counting the number of codeable labels specific for each of the different particular targets.
Disclosed is also the above method, further comprising separating any detectable complexes produced from codeable labels that are not included in the detectable complex after treating the reaction mixture and before quantitating each of the different particular targets.
Disclosed is also a method for quantitating at least two different target nucleic acid sequences in a sample comprising: forming a ligation reaction mixture by combining the sample with a different probe set specific for each of the at least two different target nucleic acid sequences, each probe set comprising (a) at least one separating bead, comprising a magnetic particle and a first target-specific probe, and (b) at least one detecting bead, comprising a codeable label, and a second target-specific probe; wherein the target- specific probes in each set are suitable for ligation together when hybridized adjacent to one another on a complementary target sequence; subjecting the ligation reaction mixture to a ligation reaction, wherein adjacent hybridizing complementary target-specific probes are ligated to one another to form a ligation product comprising the separating bead and the detecting bead; and quantitating each of the at least two different target nucleic acid sequences by counting the number of codeable labels for each different target nucleic acid sequence.
Disclosed is also the above method, further comprising separating any ligation product from unligated separating beads and detecting beads after treating the reaction mixture and before quantitating each of the at least two different target nucleic acid sequences.
Disclosed is also the above method, wherein separating the ligation product from unligated detecting and separating beads comprises: separating the ligation product from the target nucleic acid sequences, and separating the ligation product from the sample.
Disclosed is also a method for detecting at least two different target nucleic acid sequences in a sample comprising: forming a ligation reaction mixture by combining the sample with a different bead set specific for each of the at least two different target nucleic acid sequences, each bead set comprising (a) at least one separating bead, comprising a magnetic particle, a codeable label comprising at least two labels, and a first target-specific probe, wherein the first codeable label is specific for the first target-specific probe, and (b) at least one detecting bead, comprising a second codeable label comprising at least two labels and a second target-specific probe, wherein the second codeable label is specific for the second target-specific probe; wherein the first codeable label is detectably different from the second codeable label; wherein the target-specific probes in each set are suitable for ligation together when hybridized adjacent to one another on a complementary target sequence; subjecting the ligation reaction mixture to a ligation reaction, wherein adjacent hybridizing complementary target-specific probes are ligated to one another to form a detectable complex comprising the separating bead and the detecting bead; and quantitating the at least two different target nucleic acid sequences in the sample by quantitating the detectable complex.
Furthermore, disclosed is the above method, wherein the separating of the ligation product from the target nucleic acid sequences comprises thermal denaturation.
Disclosed is also the above method, further comprising removing any separating beads that are not in a ligation product prior to the quantitating the target nucleic acid sequences.
Disclosed is also the above method, wherein the removing of any separating beads that are not in a ligation product comprises: placing any separating beads and ligation products in a density gradient, wherein the separating beads and ligation products differ in density; and removing any separating beads that are not in a ligation product.
Disclosed is also the above method, wherein the codeable label has a level of intensity that is specific for the second target-specific probe.
Disclosed is also the above method, wherein the separating bead further comprises a second codeable label, and wherein the second codeable label has a level of intensity that is specific for the first target-specific probe.
Disclosed is also the above method, wherein each of the at least two probe sets that are specific for target nucleic acid sequences comprise codeable labels that have the same emission spectrum.
Disclosed is also the above method, wherein the codeable label is one or more quantum dots.
Disclosed is also the above method, wherein the codeable label is one or more quantum dots and wherein the detecting bead of each probe set comprises at least 1,000 quantum dots, wherein the quantum dots have predetermined wavelengths that make the detecting bead distinguishable from different detecting beads.
Disclosed is also the above method, wherein the separating bead further comprises at least 1,000 quantum dots, wherein the quantum dots have predetermined wavelengths that make the separating bead distinguishable from different separating beads.
Disclosed is also the above method, wherein the quantitating the at least two target nucleic acid sequences in the sample is performed in a detecting vessel comprising a groove on one surface of the detecting vessel near a magnetic source, wherein the separating bead fits in the groove, the detecting bead does not fit in the groove, and the ligation products attracted to the magnetic source are aligned.
Disclosed is also the above method, wherein the ligation reaction mixture further comprises a ligation agent. Disclosed is also the above method, wherein the ligation agent is a ligase.
Disclosed is also the above method, wherein the ligation agent is a thermostable ligase. Disclosed is also the above method, wherein the thermostable ligase is selected from at least one of Tth ligase, Taq ligase, and Pfu ligase.
Disclosed is also the above method, wherein each separating bead differs in density from each detecting bead, such that the distance between any separating beads that are not in a ligation product and the ligation product allows attraction of the ligation product to a magnetic device and does not allow attraction of the separating beads that are not in a ligation product to the magnetic device.
Disclosed is also the above method, wherein the quantitating the ligation product occurs in the presence of the sample.
Disclosed is also the above method, wherein the codeable labels comprise at least two phosphors. Disclosed is also the above method, wherein the codeable labels comprise at least two fluorescent molecules.
Disclosed is also the above method, further comprising separating the detectable complex from unligated detecting and separating beads after the ligation reaction and prior to the quantitating the at least two different target nucleic acid sequences.
Disclosed is also the above method, wherein separating the detectable complex from unligated detecting and separating beads comprises: separating the detectable complex from the at least two different target nucleic acid sequences, and separating the detectable complex from the sample. Disclosed is also the above method, wherein the separating of the detectable complex from the at least two different target nucleic acid sequences comprises thermal denaturation.
Disclosed is also the above method, further comprising removing any separating beads that are not in a detectable complex prior to the quantitating the at least two different target nucleic acid sequences.
Disclosed is also the above method, wherein the removing of any separating beads that are not in a detectable complex comprises: placing any separating beads and detectable complexes in a density gradient, wherein the separating beads and detectable complexes differ in density; and removing any separating beads that are not in a detectable complex.
Disclosed is also the above method, wherein the detecting bead further comprises a magnetic particle.
Disclosed is also the above method, wherein the quantitating the at least two target nucleic acid sequences in the sample is performed in a detection vessel comprising a groove on one surface of the detection vessel near a magnetic source, wherein the groove comprises a first end and a second end, and wherein the first codeable label and the second codeable label of the detectable complex are aligned within the groove with the magnetic source, such that the separating bead of the detectable complex aligns closer to the first end than the detecting bead of the detectable complex.
In addition, a kit for detecting target nucleic acid sequences in a sample is disclosed comprising: a different bead set specific for each of the target nucleic acid sequences, the bead set comprising (a) at least one separating bead, comprising a magnetic particle, a first codeable label comprising two or more labels, and a first target- specific probe, wherein the first codeable label is specific for the first target-specific probe, and (b) at least one detecting bead, comprising a second codeable label comprising a set of two or more labels, and a second target-specific probe, wherein the second codeable label is specific for the second target-specific probe; wherein the first codeable label is detectably different from the second codeable label ; and wherein the target-specific probes in each set are suitable for ligation together when hybridized adjacent to one another on a complementary target sequence. Disclosed is also said kit, further comprising a ligation agent. Disclosed is also said kit, wherein the ligation agent is a ligase.
Disclosed is also said kit, wherein the ligation agent is a thermostable ligase. Disclosed is also said kit, wherein the thermostable ligase is selected from at least one of Tth ligase, Taq ligase, and Pfu ligase.

## Claims

1. A kit for detecting target nucleic acid sequences in a sample comprising:
a different bead set specific for each of the target nucleic acid sequences, the bead set comprising
(a) at least one separating bead, comprising a magnetic particle, a first codeable label comprising two or more labels, and a first target-specific probe, wherein the first codeable label is specific for the first target-specific probe, and
(b) at least one detecting bead, comprising a second codeable label comprising a set of two or more labels, and a second target-specific probe, wherein the second codeable label is specific for the second target-specific probe; wherein the first codeable label is detectably different from the second codeable label; and wherein the target-specific probes in each set are suitable for ligation together when hybridized adjacent to one another on a complementary target sequence.

2. The kit of claim 1, further comprising a ligation agent.

3. The kit of claim 2, wherein the ligation agent is a ligase.

4. The kit of claim 2, wherein the ligation agent is a thermostable ligase.

5. The kit of claim 4, wherein the thermostable ligase is selected from at least one of T*th* ligase, *Taq* ligase, and *Pfu* ligase.

6. The kit of claim 1, further comprising a detecting vessel comprising a groove on one surface of the detecting vessel near a magnetic source, wherein the separating bead fits in the groove, the detecting bead does not fit in the groove.


SEQUENCE LISTING

<110> Applera Corporation

<120> Digital Assay

<130> 120-003T1

<150> EP 02797138.1 (PCT/US02/37499)
2002-11-21

<150> US 60/332,519
<151> 2001-11-21

<150> US 60/384,731
<151> 2002-05-31

<160> 11

<170> PatentIn version 3.1

<210> 1
<211> 40
<212> DNA
<213> Artificial

<220>
<223> Synthetic oligonucleotide

<400> 1
tacggatgct cactacgcta ggtttttttt tttttttttt 40

<210> 2
<211> 40
<212> DNA
<213> Artificial

<220>
<223> Synthetic oligonucleotide

<400> 2
tttttttttt tttttttttt atgcctcgtg actgctacca 40

<210> 3
<211> 80
<212> DNA
<213> Artificial

<220>
<223> Synthetic oligonucleotide

<400> 3
aaaaaaaaaa aaaaaaaacc tagcgtagtg agcatccgta tggtagcagt cacgaggcat 60

aaaaaaaaaa aaaaaaaaaa 80

<210> 4
<211> 80

<212>  DNA
<213>  Artificial

<220>
<223>  Synthetic oligonucleotide

<400>  4
tttttttttt tttttttttt atgcctcgtg actgctacca tacggatgct cactacgcta      60

ggtttttttt tttttttttt                                                  80


<210>  5
<211>  36
<212>  DNA
<213>  Artificial

<220>
<223>  Synthetic oligonucleotide

<400>  5
gctgatgcta ctggatcgct accgtgaccc ttccga                                36


<210>  6
<211>  38
<212>  DNA
<213>  Artificial

<220>
<223>  Synthetic oligonucleotide

<400>  6
gcttgcctgc tcgacttaga aatcggtctc gtccttca                              38


<210>  7
<211>  38
<212>  DNA
<213>  Artificial

<220>
<223>  Synthetic oligonucleotide

<400>  7
gcgtaatcgt tgcttcatag cctggcagta aattctag                              38


<210>  8
<211>  37
<212>  DNA
<213>  Artificial

<220>
<223>  Synthetic oligonucleotide

<400>  8
acagcagtga gtctttaggc ctggcagtaa attctac                               37


<210>  9
<211>  36

<212>  DNA
<213>  Artificial

<220>
<223>  Synthetic oligonucleotide

<400>  9
ctatgaagca acgattacgc tcggaagggt cacggt                                36


<210>  10
<211>  37
<212>  DNA
<213>  Artificial

<220>
<223>  Synthetic oligonucleotide

<400>  10
cctaaagact cactgctgtt gaaggacgag accgatt                               37


<210>  11
<211>  38
<212>  DNA
<213>  Artificial

<220>
<223>  Synthetic oligonucleotide

<400>  11
ctcaacctta cttgaggctg gtagcagtca cgaggcat                              38